# EUROPEAN PATENT APPLICATION

(11) **EP 3 103 454 A1**
(43) Date of publication of application: **14.12.2016**
(21) Application number: 16180014.9
(22) Date of filing: 05.04.2012
(51) Int. Cl.: A61K 31/522, A61K 31/40, A61K 31/403, A61K 45/06, A61K 9/00, A61P 9/00, A61P 9/04, A61P 9/10, A61K 47/06, A61K 9/02

(54) **USE OF A2B ADENOSINE RECEPTOR ANTAGONISTS FOR TREATING HEART FAILURE AND ARRHYTHMIA IN POST-MYOCARDIAL INFARCTION PATIENTS**

(30) Priority: 07.04.2011 US 201161473110 P; 07.06.2011 US 201161494222 P; 21.12.2011 US 201161578728 P; 30.03.2012 US 201261618581 P
(62) Divisional of application: 12715516.6
(71) Applicant: Gilead Sciences, Inc., Foster City, CA 94404 (US)
(72) Inventor: BELARDINELLI, Luiz, Palo Alto CA 94306 (US); ZHONG, Hongyan, Mountain View CA 94040 (US); ZENG, Dewan, Palo Alto CA 94303 (US)
(74) Representative: Lumsden, Stuart Edward Henry

(57) **Abstract**

Provided are methods of improving the cardiac condition of post-myocardial infarction (MI) patients and reducing cardiovascular death and hospitalization due to heart failure or arrhythmias, by administering a therapeutically effective amount of an A_{2B} adenosine receptor antagonist.

## Description

### CROSS REFERENCE TO RELATED APPLICATIONS

This application claims the benefit under 35 U.S.C. § 119(e) of United States Provisional Applications Serial Number 61/473,110 filed April 7, 2011, Serial Number 61/494,222 filed June 7, 2011, Serial Number 61/578,728 filed December 21, 2011, and Serial Number 61/618,581 filed March 30, 2012, the content of each of which is incorporated by reference in its entirety into the present disclosure.

### FIELD OF THE DISCLOSURE

The disclosure is directed to methods of improving the cardiac function in patients after myocardial infarction (MI) and to reduce cardiovascular death and hospitalization due to heart failure or arrhythmias. The methods comprise administering a therapeutically effective amount of an A_{2B} adenosine receptor antagonist.

### BACKGROUND

Acute myocardial infarction (AMI) is characterized by ischemic necrosis of the myocardium followed by an intense inflammatory response. The extent of the initial loss of viable myocardium and the intensity of the inflammatory response are both independent predictors of the ensuing cardiac remodeling characterized by cardiac enlargement and dysfunction.

Myocardial injury can induce cardiac fibrosis leading to arrhythmias and heart failure. Each year about 200,000 people suffer acute STEMI (ST segment elevation myocardial infarction), which has about a 20% mortality within one year. In 2011, there were about 7.6 million post-myocardial infarction patients, and it was estimated that 10-70% of them progress to heart failure within 5 years.

Not all cardiac fibrosis following myocardial infarction (MI) is harmful, however. For instance, immediately following the AMI, tissues in the infarct zone undergo a compensatory fibrotic repair of the necrotic area with scar formation. Without the proper fibrotic repair and scar, the heart may rupture, which could be fatal.

Pathologic LV remodeling during the maladaptive phase, in contrast, can lead to heart failure and arrhythmia. Therefore, blind inhibition of cardiac fibrosis, without the correct timing or location, would not be effective in restoring the cardiac function and reduce death of post-MI patients. Rather, such treatments can lead to severe adverse events.

Interstitial fibrosis impede the normal coupling of cardiac myocytes and create a substrate for arrhythmia, which could lead to sudden cardiac death.

Cardiac fibrosis has been associated with the heart failure process leading to a variety of heart diseases, including those associated with both volume and pressure overload (Weber et al., Circ., 83:1849-1865 (1991); Schaper et al., Basic Res. Cardiol., 87:S1303-S1309 (1992); Boluyt et al., Circ. Res., 75:23-32 (1994); and Bishop et al., J. Mol. Cell Cardiol., 22:1157-1165 (1990)). In the case of heart failure, fibrosis involves an increase in both fibroblast number and matrix deposition (Cardiovasc. Res., 30:537-543 (1995)), suggesting the importance of the fibroblast in the development of this condition.

Adenosine (Ado) is a ubiquitous small molecule released in response to tissue injury that promotes hyperemia and modulates inflammation through interaction with one of four types of cell surface membrane receptors. In some models of inflammation blockade of the A_{2B} AdoR has limited the intensity of the inflammatory response and improved healing, whereas in others A_{2B} AdoR signaling appeared to inhibit inflammation. Thus, one could not generalize the role of A_{2B} AdoR.

### SUMMARY

This disclosure is directed to the surprising and unexpected discovery that A_{2B} adenosine receptor antagonists inhibit pathologic left ventricular (LV) remodeling and increase LV ejection fraction. In post-myocardial infarction (MI) patients, inhibition of LV remodeling may translate into an improvement in LV function and fewer incidence of arrhythmias, which in turn can reduce cardiovascular (CV) death and hospitalization.

It has also been discovered that the A_{2B} adenosine receptor (AdoR) is the predominant subtype of AdoRs expressed in human cardiac fibroblasts (HCF) and activation of this receptor increases the release of IL-6 and production of collagen, expression of fibrotic markers and release of biomarkers of cardiovascular diseases (CVD). In particular, it has been discovered that an A_{2B} adenosine receptor antagonist could completely abolish such activation, thereby reducing fibrotic response in heart diseases, leading to inhibition of LV enlargement and increased LV ejection fraction.

In light of the above and in one of its method aspects, the disclosure is directed to a method of reducing progression of heart failure and/or reducing the incidence of arrhythmia and/or sudden cardiac death in a patient that has suffered myocardial infarction (MI), comprising administering to the patient a therapeutically effective amount of an A_{2B} adenosine receptor antagonist. In some embodiments, death or hospitalization is reduced by treatment of the heart failure or arrhythmia.

Another embodiment provides a method of reducing the progression of heart failure, reducing or treating heart failure in a patient that has suffered myocardial infarction (MI), comprising administering to the patient a therapeutically effective amount of an A_{2B} adenosine receptor antagonist.

Another embodiment provides a method of reducing the incidence of arrhythmia in a patient that has suffered myocardial infarction (MI), comprising administering to the patient a therapeutically effective amount of an A_{2B} adenosine receptor antagonist.

Another embodiment provides a method of reducing the incidence of sudden cardiac death in a patient that has suffered myocardial infarction (MI), comprising administering to the patient a therapeutically effective amount of an A_{2B} adenosine receptor antagonist.

Another embodiment provides a method of increasing the left ventricle ejection fraction (LVEF) in a patient that has suffered myocardial infarction (MI), comprising administering to the patient a therapeutically effective amount of an A_{2B} adenosine receptor antagonist.

Another embodiment provides a method of inhibiting left ventricle enlargement in a patient that has suffered myocardial infarction (MI), comprising administering to the patient a therapeutically effective amount of an A_{2B} adenosine receptor antagonist.

Another embodiment provides a method of reducing left ventricle end systolic volume in a patient that has suffered myocardial infarction (MI), comprising administering to the patient a therapeutically effective amount of an A_{2B} adenosine receptor antagonist.

Another embodiment provides a method of reducing left ventricle end diastolic volume in a patient that has suffered myocardial infarction (MI), comprising administering to the patient a therapeutically effective amount of an A_{2B} adenosine receptor antagonist.

Another embodiment provides a method of ameliorating left ventricle dysfunction in a patient that has suffered myocardial infarction (MI), comprising administering to the patient a therapeutically effective amount of an A_{2B} adenosine receptor antagonist.

Another embodiment provides a method of improving myocardial contractibility in a patient that has suffered myocardial infarction (MI), comprising administering to the patient a therapeutically effective amount of an A_{2B} adenosine receptor antagonist.

Another embodiment provides a method of reducing the release of IL-6, TNFα, ST2 (suppression of tumorigenicity 2) or BNP from a cardiac cell in a patient that has suffered myocardial infarction (MI), comprising administering to the patient a therapeutically effective amount of an A_{2B} adenosine receptor antagonist.

In one embodiment, the A_{2B} adenosine receptor antagonist is a 8-cyclic xanthine derivative. In another embodiment, the A_{2B} adenosine receptor antagonist is a compound of Formula I or II: 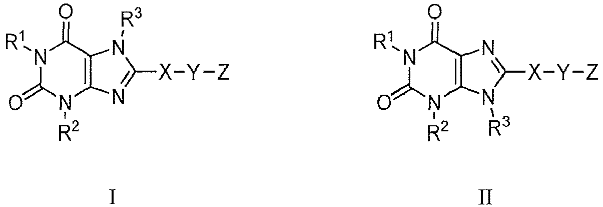 wherein:
- R¹ and R²: are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl or optionally substituted alkynyl;
- R³: is hydrogen, optionally substituted alkyl or optionally substituted cycloalkyl;
- X: is optionally substituted arylene or optionally substituted heteroarylene;
- Y: is a covalent bond or alkylene in which one carbon atom can be optionally replaced by-O-, -S-, or -NH-, and is optionally substituted by hydroxy, alkoxy, optionally substituted amino, or -COR, in which R is hydroxy, alkoxy or amino;
- Z: is optionally substituted monocyclic aryl or optionally substituted monocyclic heteroaryl; or
- Z: is hydrogen when X is optionally substituted heteroarylene and Y is a covalent bond;
- or: a pharmaceutically acceptable salt, tautomer, isomer, a mixture of isomers, or prodrug thereof.

In a particular aspect, the A_{2B} adenosine receptor antagonist is N-(6-amino-1-ethyl-2,4-dioxo-3-propyl-1,2,3,4-tetrahydro-pyrimidin-5-yl)-1-(3-(trifluoromethyl)benzyl)-1H-pyrazole-4-carboxamide, referred to throughout as Compound A, having the following chemical formula: 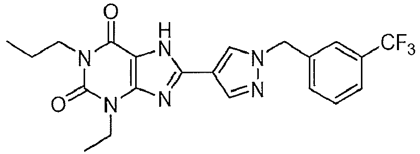 or a pharmaceutically acceptable salt, tautomer, isomer, or a mixture of isomers thereof. In some aspects, the A_{2B} adenosine receptor antagonist is Compound B or C, as further described herein.

### BRIEF DESCRIPTION OF THE DRAWINGS

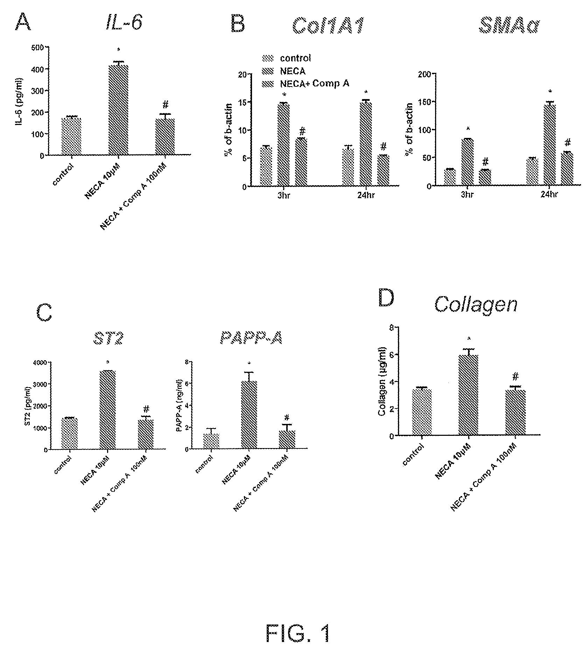

**FIG. 1A-D** show that the effects of NECA on release of IL-6, collagen, ST-2 and PAPPA and on expression of α-smooth muscle actin (SMAα) and α-1 pro-collagen (Col1A1) were completely abolished by a selective A_{2B} AdoR antagonist, Compound A (Comp A).

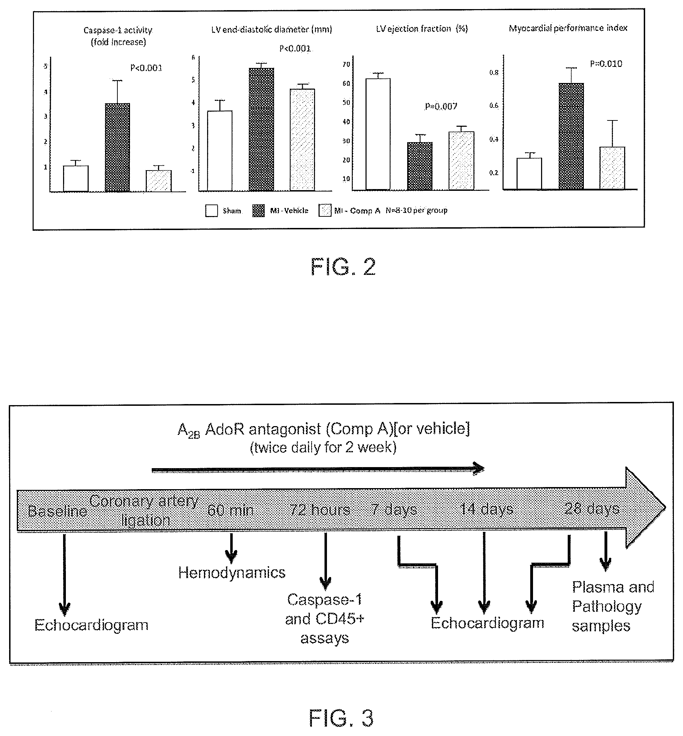

**FIG. 2** shows that treatment with Compound A (Comp A) significantly reduced caspase-1 activity (left panel), reduced the end-diastolic diameter (left middle panel), increased LV ejection fraction (right middle panel) and reduced myocardial performance index (right panel) compared to vehicle following acute myocardial infarction in mice.
**FIG. 3** is the timeline for the study in Example 4.

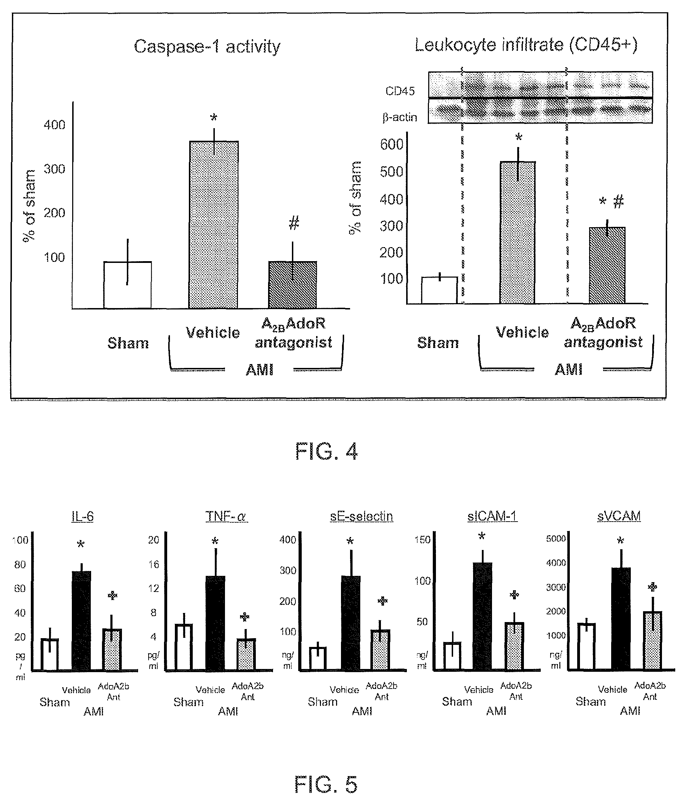

**FIG. 4** shows that administration of the A_{2B} AdoR antagonist, Compound A (Comp A), given immediately after coronary ligation (4 mg/kg i.p. twice daily) prevented caspase-1 activation in the heart tissue measured 72 hours after surgery and significantly inhibited leukocyte (CD45+) recruitment in the heart. * P<0.001 vs sham; # P<0.01 vs vehicle. N=4-6 per group.
**FIG. 5** includes charts to show that treatment with the A_{2B} AdoR antagonist, Compound A, led to a significant reduction in plasma levels of inflammatory markers associated with AMI Plasma concentrations of IL-6, TNF-α, sE-selectin, sICAM, and sVCAM were measured using Luminex at day 28 after surgery. + P<0.05 vs vehicle; * P<0.001 vs vehicle.

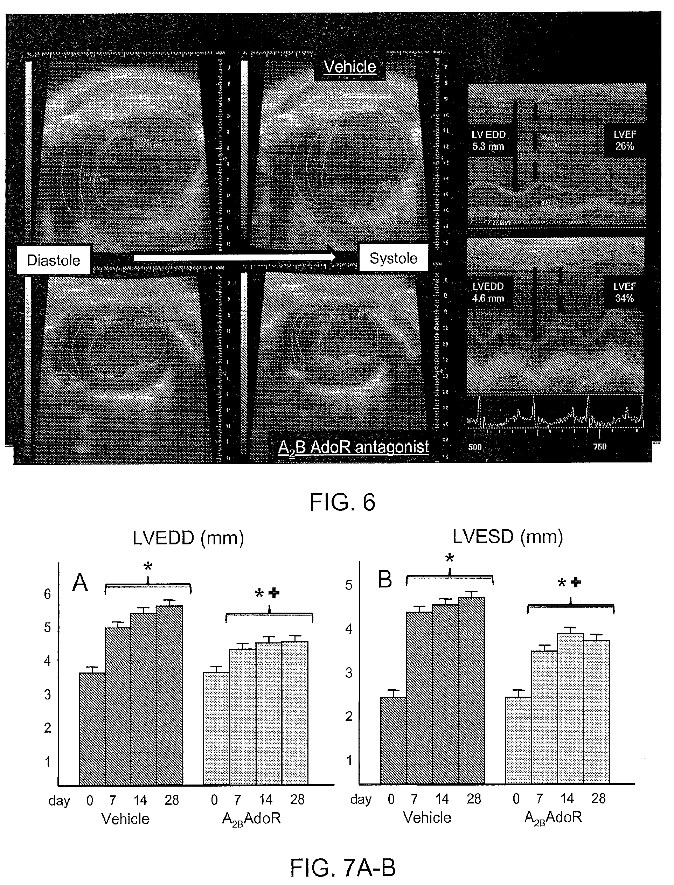

**FIG. 6** presents exemplary B-Mode and M-Mode echocardiographic images from a mouse treated with vehicle (top panels) and one treated with the A_{2B} AdoR antagonist, Compound A (bottom panels) 2 weeks after acute myocardial infarction surgery.
**FIG. 7A-F** show that, following coronary artery ligation surgery, vehicle-treated mice had a significant enlargement of the left and right ventricles (left ventricular end-diastolic diameter (LVEDD) **(A),** left ventricular end-systolic diameter (LVESD) **(B)** and right ventricular end-diastolic area (RVEDA)) **(E)** and a significant reduction in left and right ventricular function (left Ventricular ejection fraction (LVEF) **(C),** myocardial performance index (MPI) **(D)** and tricuspidal annular plane systolic exercusion (TAPSE)) (F). Treatment with the A_{2B} AdoR antagonist, Compound A (4 mg i.p. twice daily) significantly limited the cardiac enlargement and dysfunction. *P<0.001 vs baseline (or sham), + P<0.001 vs vehicle.

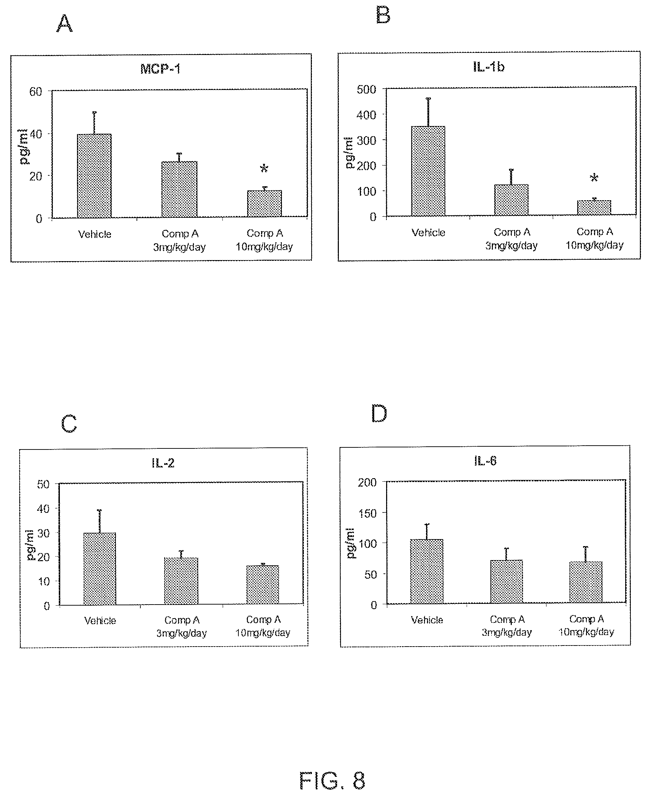

**FIG. 8A-D** show that expression of four inflammatory biomarkers, MCP-1, IL-1b, IL-2 and IL-6, were reduced by treatment with Compound A and for two of them, MCP-1 and IL-1b, the reduction in the 10 mg/kg/day group was statistically significant.

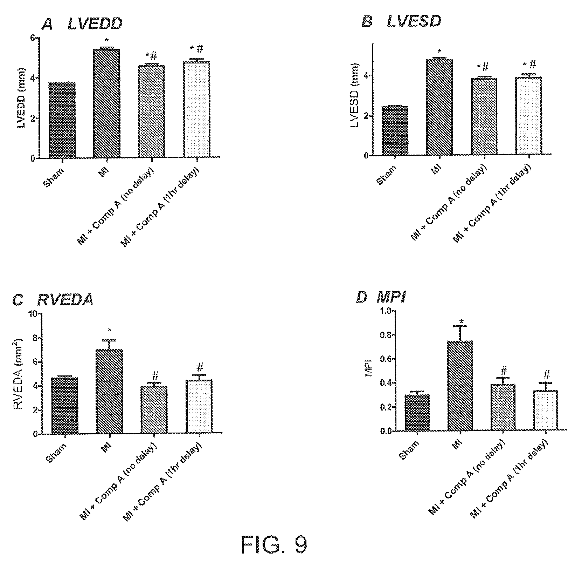

**FIG. 9A-D** show LVEDD **(A),** LVESD **(B),** RVEDA **(C)** and MPI **(D)** measured using ECHO⁶ on Day 28. Values are presented as mean ± SEM. n = 4-9. *, p < 0.05 compared to sham control; #, p < 0.05 compared to MI. Statistics were determined using ANOVA followed by Bonferroni test. These figures demonstrate that Compound A inhibits adverse post-MI myocardial remodeling and improves myocardial function.

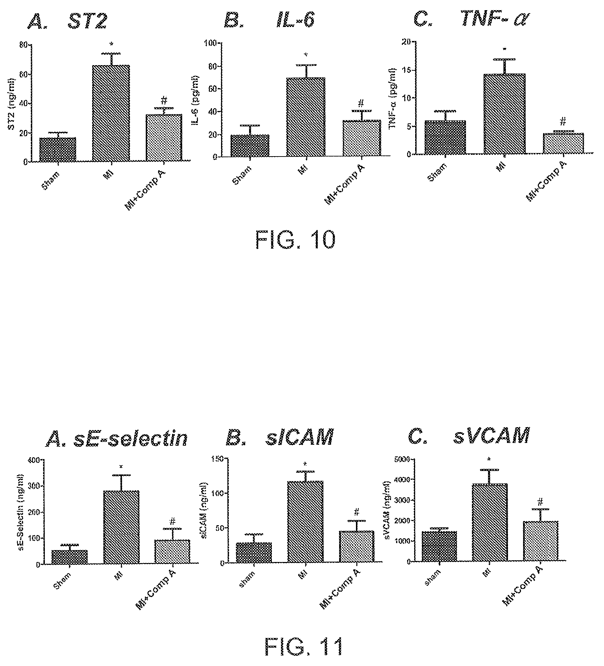

**FIG. 10A-C** show plasma levels of ST2 (A) IL-6 (B) and TNF-α (C) and values are presented as mean ± SEM. n = 4-8. *, p < 0.05 compared to sham control; #, p < 0.05 compared to MI. Statistics were determined using ANOVA followed by Bonferroni test. These figures show that Compound A reduces plasma inflammatory cytokine biomarkers in a mouse model of post-MI myocardial remodeling
**FIG. 11A-C** present plasma levels of sE-selectin **(A),** sICAM **(B),** and sVCAM (C). Values are presented as mean ± SEM. n = 4. *, p < 0.05 compared to sham control; #, p < 0.05 compared to MI. Statistics were determined using ANOVA followed by Bonferroni test. These figures show that Compound A reduces plasma levels of soluble adhesion molecules in a mouse model of post-MI myocardial remodeling.

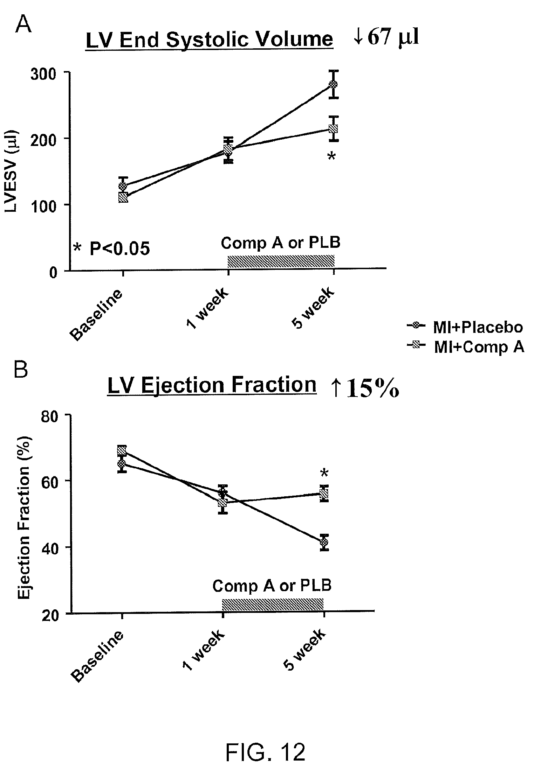

**FIG. 12A-B** include charts to show that Compound A reduced left ventricular end-systolic volume (A) and increased left ventricle ejection fraction **(B).** (A) left ventricular end-systolic volume (LVESV) in MI and MI + Compound A group was measured using ECHO at baseline, 1 week and 5 weeks post-MI. Values are presented as mean ± SEM. n =13. *, p<0.05 compared to vehicle control using ANOVA followed by Bonferroni test. **(B)** LVEF in vehicle control (MI) and Compound A group (MI + Compound A) was measured using ECHO at baseline, 1 week and 5 weeks post-MI. LVEF was calculated using the formula: (LVEDV - LVESV)/LVEDV * 100. Values are presented as mean ± SEM. n = 13. *, p<0.05 compared to vehicle control using ANOVA followed by Bonferroni test.

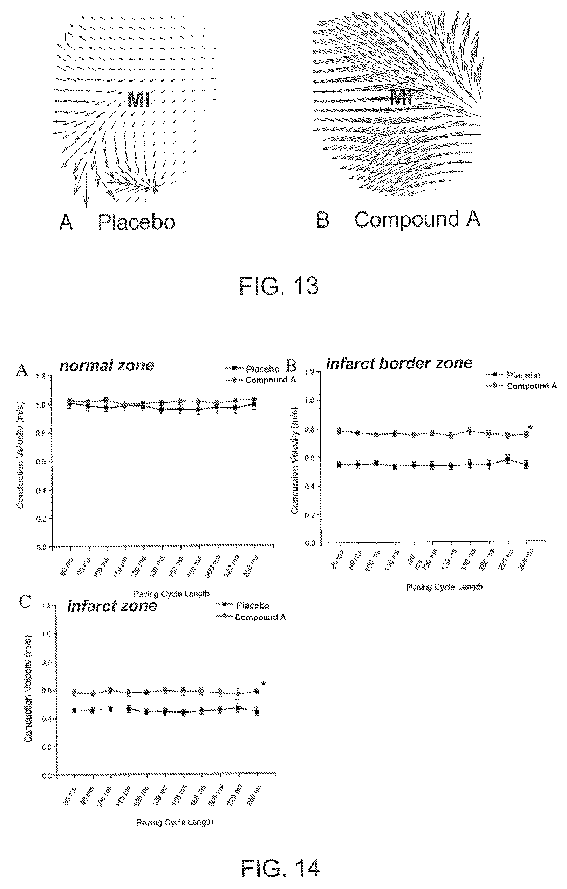

**FIG. 13A-B** are conduction vector maps of LV anterior walls showing conduction velocity Smaller arrows near designated MI areas more notable in the vehicle control **(A)** as compared with the Compound A sample **(B).**
**FIG. 14A-C** show that Compound A increases conduction velocities in infarct zones and infarct border zones (IBZs). CVs for normal **(A),** border **(B),** and infarct **(C)** zones in Placebo Control and Compound A-treated MI rats at various pacing cycle lengths. **, p* < 0.05 compared to placebo control.

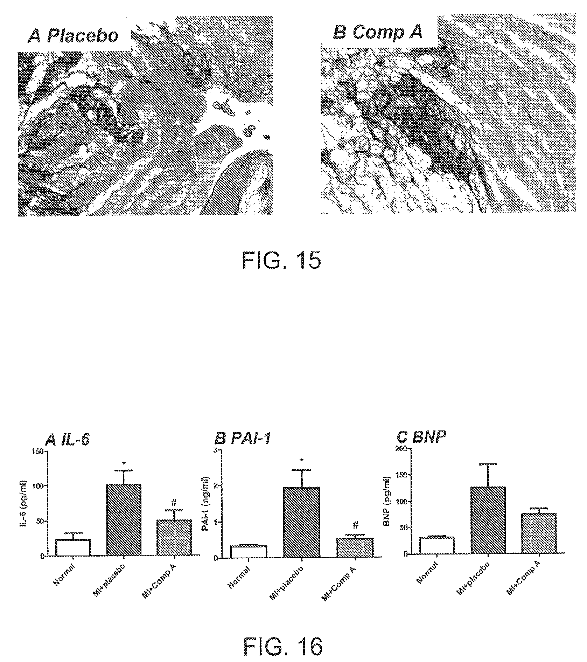

**FIG. 15A-B** are high-powered microscopy (100x) images of IBZs stained with Sirius red (shown as dark gray) for fibrosis with fast green counter-stain (light gray). Fibrosis extended in a finger-like distribution from the infarct area into border areas for the vehicle control **(A),** but to a lesser extent for the Compound A sample **(B).** These images show that Compound A reduces fibrosis in the IBZ.
**FIG. 16A-C** show plasma levels of IL-6 (A), PAI-1 (B), and BNP (C) measured using luminex at 5 week post-MI. Values are presented as mean ± SEM. n = 4-8. *, p < 0.05 compared to normal control; #, p < 0.05 compared to MI. Statistics were determined using ANOVA followed by Bonferroni test. These figures show that Compound A reduces plasma biomarkers in rat model of post-MI remodeling.

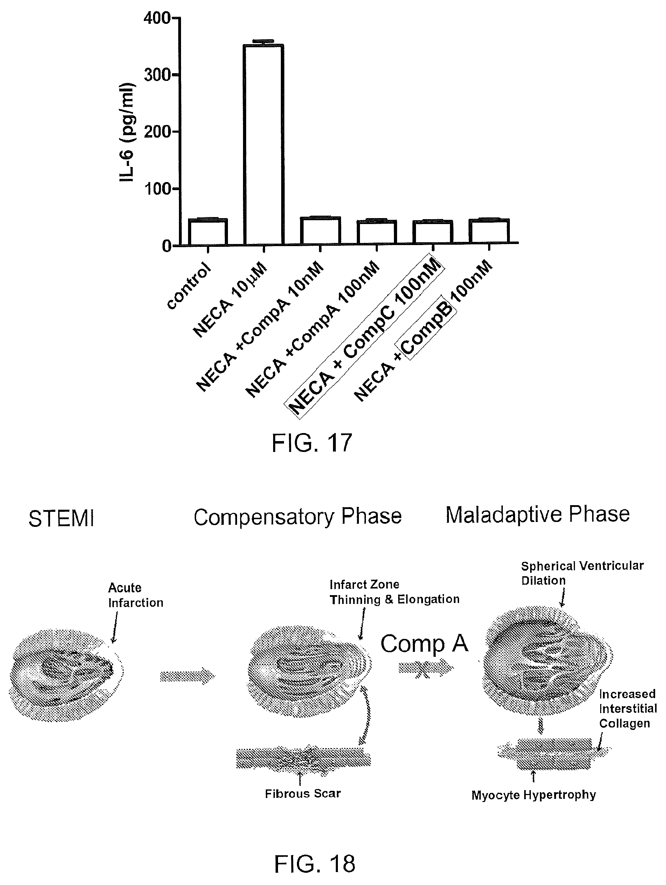

**FIG. 17** is a chart showing the IL-6 release from human cardiac myocytes treated with NECA, along with Compound A, B, or C, at indicated doses.
**FIG. 18** illustrates a proposed mechanism by which Compound A inhibits pathologic post-MI LV remodeling and improves cardiac functions.

### DETAILED DESCRIPTION

Prior to describing this disclosure in greater detail, the following terms will first be defined.

It is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

It must be noted that as used herein and in the appended claims, the singular forms "a", "an", and "the" include plural referents unless the context clearly dictates otherwise.

### 1. Definitions

Unless defined otherwise, all technical and scientific terms used herein have the same meaning as commonly understood by one of ordinary skill in the art to which this disclosure belongs. As used herein the following terms have the following meanings.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed disclosure. "Consisting of' shall mean excluding more than trace elements of other ingredients and substantial method steps. Embodiments defined by each of these transition terms are within the scope of this disclosure.

The term "about" when used before a numerical designation, *e.g.,* temperature, time, amount, and concentration, including range, indicates approximations which may vary by (+) or (-) 10 %, 5 % or 1 %.

The term "treatment" means any treatment of a disease in a patient including: (i) preventing the disease, that is causing the clinical symptoms not to develop; (ii) inhibiting the disease, that is, arresting the development of clinical symptoms; and/or (iii) relieving the disease, that is, causing the regression of clinical symptoms. By way of example only, treating may include improving right ventricular function and/or alleviating symptoms, including, but not limited to exertional dyspnea, fatigue, chest pain, and combinations thereof.

As used herein, the term "ameliorate", when used in reference to the severity of a pathologic condition, means that signs or symptoms associated with the condition are lessened, or improvement in the subject's condition. The signs or symptoms to be monitored will be characteristic of a particular pathologic condition and will be well known to skilled clinician, as will the methods for monitoring the signs and conditions.

The term "patient" typically refers to a mammal, such as, for example, a human.

The term "therapeutically effective amount" refers to that amount of a compound, such as an A_{2B} adenosine receptor antagonist, that is sufficient to effect treatment, as defined above, when administered to a patient in need of such treatment. The therapeutically effective amount will vary depending upon the specific activity or delivery route of the agent being used, the severity of the patient's disease state, and the age, physical condition, existence of other disease states, and nutritional status of the patient. Additionally, other medication the patient may be receiving will affect the determination of the therapeutically effective amount of the therapeutic agent to administer.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

### 2. Methods

As stated above, the present disclosure relates to methods of treating heart failure and/or arrhythmia in a patient that has suffered myocardial infarction (MI). In one embodiment, the methods described herein may reduce death or hospitalization in patients by treating the heart failure or arrhythmia. Moreover, such methods achieve improved cardiac condition of post-myocardial infarction (MI) patients. The methods entail administering to a patient in need thereof a therapeutically effective amount of an A_{2B} adenosine receptor antagonist.

It has been discovered herein that the A_{2B} adenosine receptor (AdoR) is the predominant subtype of AdoRs expressed in human cardiac fibroblasts (HCF), and activation of this receptor increases the release of IL-6 and production of collagen, release of fibrotic markers and release of biomarkers of cardiovascular diseases (CVD).

Further, N-ethylcarboxamide adenosine (NECA), a stable analog of adenosine, significantly increased the release of IL-6 in a concentration-dependent manner. Moreover, NECA increased the expression of α-mooth muscle actin and α-1 pro-collagen, increased the production of collagen from HCF, and increased the release of two novel biomarkers of CVD, soluble ST-2 and PAPPA (Pregnancy-associated plasma protein A). The effects of NECA on HCF, however, were completely abolished by an A_{2B} AdoR antagonist (Example 2).

At a physiological level, adenosine levels rise in ischemic myocardium. The increased adenosine levels then activate A_{2B} receptors on macrophages, cardiac myocytes and cardiac fibroblasts, resulting in release of inflammatory and fibrotic mediators contributing to left ventricular dysfunction. An A_{2B} AdoR antagonist's ability to inhibit such activation, in turn, can lead to inhibition of the release of such inflammatory and fibrotic mediators. Also, an A_{2B} AdoR antagonist can inhibit macrophage activation in injured cardiac tissues. In sum, the A_{2B} adenosine receptor antagonists of the present disclosure are shown to be able to reduce fibrotic response in heart diseases, leading to improved left ventricular function.

Consistent with these mechanistic findings, it has now been observed, in rat and mouse ST segment elevation myocardial infarction (STEMI) models, that A_{2B} adenosine receptor antagonists inhibited left ventricular (LV) enlargement, as shown with decreased enlargement of LV end systolic volume and end diastolic volume, and increased LV ejection fraction. As inhibition of LV enlargement leads to improved LV function and inhibition of fibrosis in the infarct border zone (IBZ) leads to fewer arrhythmias, the A_{2B} adenosine receptor antagonists can reduce cardiovascular death and hospitalization due to heart failure or arrhythmias. In this respect, it is contemplated that interstitial fibrosis creates a substrate for anhythmia. By reducing interstitial fibrosis, therefore, the A_{2B} adenosine receptor antagonists can reduce the incidence of arrhythmia and sudden cardiac death.

An unexpected aspect of these findings is that the activities of the A_{2B} adenosine receptor antagonists do not interfere with the post-MI compensatory fibrotic response in the infarct zone. As illustrated in **FIG. 18****,** during the acute phase of myocardial infarction (*e.g.,* STEMI), cells in the infarct zone suffer necrosis. Following the acute phase (*e.g.*, about one week following the MI), the infarct zone thins and elongates to compensate for the loss due to fibrous scar from the necrotic cells. Such a compensatory mechanism (also known as LV dilation) is beneficial to maintaining the function of the LV.

Further enlargement of the LV during the maladaptive phase, however, can lead to heart failure and arrhythmia. In this respect, the LV enlargement at this phase occurs at areas outside the infarct zone resulting in spherical ventricular dilation. Further, the enlarged LV leads to LV dysfunction characterized with decreased ejection fraction.

Surprisingly, the A_{2B} adenosine receptor antagonists of the present disclosure specifically inhibit, in cardiac tissues outside the infarct zone, the release of inflammatory and fibrotic mediators, reducing inflammation and fibrosis-induced tissue injury and improving ejection fraction and LV function. Therefore, contrary to the conventional perception regarding adenosines' cardiac protective role, the experimental data of the present disclosure demonstrate the therapeutic effect of the inhibition of adenosines' activities, with A_{2B} adenosine receptor antagonists, in post-MI cardiac protection and recovery.

Therefore, when used in post-MI patients, A_{2B} adenosine receptor antagonists can be administered to the patient even before the compensatory cardiac fibrosis is over, not risking inhibiting such a compensatory response. Moreover, it is contemplated that the post-MI patients are hemodynamically stable when receiving the treatment. Further, it is contemplated that the antagonists may be administered as early as during the MI or immediately following the MI.

Also surprising is the extent of the therapeutic effect of A_{2B} adenosine receptor antagonists. For instance, when compared to other anti-fibrotic drugs, such as pirfenidone, which only slowed the reduction of LVEF, the A_{2B} adenosine receptor antagonists of the present disclosure were able to halt or even reverse such reduction (Example 6 and **FIG. 12B****).**

Moreover, such effects of A_{2B} adenosine receptor antagonists are observed with multiple compounds, including Compound A, B (defined below) and C (defined below), demonstrating that A_{2B} adenosine receptor antagonists, in general, possess such therapeutic capabilities. This is further illustrated in Example 7. The assays used herein, in addition to in vivo and clinical studies, can further used to determine the selectivity and PK profiles of these or other A_{2B} adenosine receptor antagonists for their suitability for clinical use.

Thus, the present disclosure provides methods for treating heart failure and/or arrhythmia in post-myocardial infarction (MI) patients. It is contemplated that by treating the heart failure and/or arrhthymia, cardiovascular death and hospitalization is reduced. Also provided are methods for reducing the risk or progression of heart failure or reducing heart failure, reducing or ameliorating arrhythmia, increasing the left ventricle ejection fraction (LVEF), inhibiting left ventricle enlargement, reducing left ventricle end systolic volume, reducing left ventricle end diastolic volume, ameliorating left ventricle dysfunction, improving myocardial contractibility or reducing cardiac fibrosis. In some aspects, the provided methods treat cardiac fibrosis in post-MI patient, whereas the treatment achieves targeted reduction of fibrosis at regions beyond the infarct zone and reduction of fibrosis and improved recovery after the compensatory fibrotic response stage. In some embodiments, reducing, increasing, improving, ameliorating, or inhibiting, as used here, is as compared to a similarly situated patient not receiving administration of the A_{2B} adenosine receptor antagonist.

In one embodiment, the methods comprise administering a therapeutically effective amount of an A_{2B} adenosine receptor antagonist to the patient.

In one aspect, the MI is acute MI. In another aspect, the MI is ST elevation MI (STEMI). In yet another aspect, the MI is non-ST elevation MI (NSTEMI).

In one aspect, administration of the A_{2B} adenosine receptor antagonist starts as early as during the MI or immediately following MI. In some embodiments, administration occurs when the patient diagnosed to suffer from MI. In another aspect, the administration starts about 1 hour, or alternatively about 2, 3, 4, 5, 6, 7, 8, 9, 10, 11, 12, 13, 14, 15, 16, 17, 18, 19, 20, 21, 22, 23, or 24 hours following the MI. In yet another aspect, the administration starts about 1 day, or alternatively about 2, 3, 4, 5, 6, 7, 8, 9, 10, 11, 12, 13 or 14 days or 1, 2, 3, or 4 weeks following the MI. In a particular aspect, the administration starts as soon as the MI patient is stabilized. In one aspect, the administration starts within one day post-MI. In another aspect, the administration starts between one and five days post-MI. In the event immediate administration is not feasible, the current data show that administration that starts one or two weeks post-MI is still effective.

In some aspects, the administration starts no later than about 3 days, or alternatively 5 days, 7 days, 2 weeks, 3 weeks or 4 weeks following the MI.

In some aspects, the post-MI patients are hemodynamically stable. Methods and criteria of determining hemodynamic stability are known in the art. The term "hemodynamically stable" as used herein refers to the restoration of one or more or all measured hemodynamic parameter to its normal range. Examples of such normal ranges are provided in the table below.

| | **Typical Value** | **Normal Range** |
|---|---|---|
| **End-diastolic volume** | 120 mL | 65 - 240 mL |
| **End-systolic volume** | 50 mL | 16 - 143 mL |
| **Stroke volume** | 70 mL | 55 - 100 mL |
| **Ejection fraction** | 0.58 | 55% - 70% |
| **Heart rate** | 75 BPM | 60 - 100 BPM |
| **Cardiac output** | 5.25 L/min | 4 - 8 L/min |

As more thoroughly described below, the antagonists may be administered in a variety of ways, including, systemic, oral, intravenous, intramuscular, intraperitoneal, and inhalation.

In another of its method aspects, this disclosure is directed to a method of inhibiting overexpression of α-smooth muscle actin and/or α-1 pro-collagen in human cardiac fibroblasts which method comprises contacting these cells with an effective amount of an A_{2B} adenosine receptor antagonist.

In yet another of its method aspects, this disclosure is directed to a method of reducing IL-6, collagen, ST-2, PAPPA (Pregnancy-associated plasma protein A) and/or caspase-1 expression and/or release from human cardiac fibroblasts which method comprises contacting these cells with an effective amount of an A_{2B} adenosine receptor antagonist.

### 3. A_{2B} Adenosine Receptor Antagonists

The term "A_{2B} adenosine receptor" or "A_{2B} receptor" refers to a subtype of an adenosine receptor. Other subtypes include A₁, A_{2A} and A₃.

The term "A_{2B} adenosine receptor antagonist" or "A_{2B} receptor antagonist" refers to any compound, peptide, protein (e.g., antibody), siRNA that inhibits or otherwise modulates the activity of the A_{2B} adenosine receptor. In one embodiment, the antagonist selectively inhibits the A_{2B} receptor over the other subtypes of adenosine receptor. In another embodiment the antagonist is partially selective for the A_{2B} receptor. Compounds that are putative antagonists may be screened using the procedure in Example 1. Compounds that are suitable for the methods of the present disclosure can be screened using experiments as illustrated in Examples 2-7. For instance, if a compound reduces NECA's induced IL-6 release, then this compound is considered suitable for the purpose of the disclosure. In one aspect, the reduction is at least about 10%, 20%, 30%, 40%, or 50%. Examples of antagonists include, but not limited to, those discussed in the section below.

A variety of A_{2B} adenosine receptor antagonists are contemplated to be useful in this invention. The compounds are described in U.S. Patent 6,825,349, 7,105,665, and 6,997,300, which are all incorporated by reference in their entirety. In one embodiment, the invention is directed to use of a compound of Formula I or II, 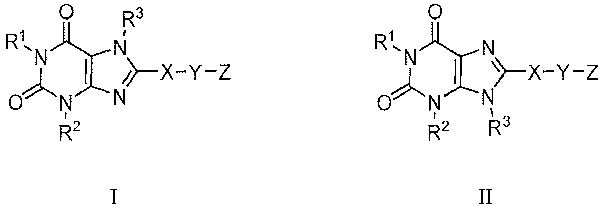 wherein:
- R¹ and R²: are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl or optionally substituted alkynyl;
- R³: is hydrogen, optionally substituted alkyl or optionally substituted cycloalkyl;
- X: is optionally substituted arylene or optionally substituted heteroarylene;
- Y: is a covalent bond or alkylene in which one carbon atom can be optionally replaced by -O-, -S-, or -NH-, and is optionally substituted by hydroxy, alkoxy, optionally substituted amino, or -COR, in which R is hydroxy, alkoxy or amino;
- Z: is optionally substituted monocyclic aryl or optionally substituted monocyclic heteroaryl; or
- Z: is hydrogen when X is optionally substituted heteroarylene and Y is a covalent bond;
- or: a pharmaceutically acceptable salt, tautomer, isomer, a mixture of isomers, or prodrug thereof.

For Formula I or II, in some embodiments,
- R¹ and R²: are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl or optionally substituted alkynyl, with the proviso that when D is a covalent bond E cannot be alkoxy;
- R³: is hydrogen, optionally substituted alkyl or optionally substituted cycloalkyl;
- X: is optionally substituted arylene or optionally substituted heteroarylene;
- Y: is a covalent bond or alkylene in which one carbon atom can be optionally replaced by -O-, -S-, or -NH-, and is optionally substituted by hydroxy, alkoxy, optionally substituted amino, or -COR, in which R is hydroxy, alkoxy or amino;
with the proviso that when the optional substitution is hydroxy or amino it cannot be adjacent to a heteroatom; and
Z is optionally substituted monocyclic aryl or optionally substituted monocyclic heteroaryl; or
Z is hydrogen when X is optionally substituted heteroarylene and Y is a covalent bond;
with the proviso that when X is optionally substituted arylene, Z is optionally substituted monocyclic heteroaryl
or a pharmaceutically acceptable salt, tautomer, isomer, a mixture of isomers, or prodrug thereof.

In one embodiment, compounds of Formula I and II are those in which R¹ and R² are independently hydrogen, optionally substituted lower alkyl, or a group -D-E, in which D is a covalent bond or alkylene, and E is optionally substituted phenyl, optionally substituted cycloalkyl, optionally substituted alkenyl, or optionally substituted alkynyl, particularly those in which R³ is hydrogen.

Within this group, a class of compounds include those in which R¹ and R² are independently lower alkyl optionally substituted by cycloalkyl, preferably n-propyl, and X is optionally substituted phenylene. Within this class, a subclass of compounds are those in which Y is alkylene, including alkylene in which a carbon atom is replaced by oxygen, preferably -O-CH₂-, more especially where the oxygen is the point of attachment to phenylene. Within this subclass, in one embodiment, Z is optionally substituted oxadiazole, particularly optionally substituted [1,2,4]-oxadiazol-3-yl, especially [1,2,4]-oxadiazol-3-yl substituted by optionally substituted phenyl or by optionally substituted pyridyl.

Another class of compounds include those in which X is optionally substituted 1,4-pyrazolene. Within this class, a subclass of compounds are those in which Y is a covalent bond, alkylene, lower alkylene, and Z is hydrogen, optionally substituted phenyl, optionally substituted pyridyl or optionally substituted oxadiazole. Within this subclass, one embodiment includes compounds in which R¹ is lower alkyl optionally substituted by cycloalkyl, and R² is hydrogen. Another embodiment includes those compounds in which Y is -(CH₂)- or -CH(CH₃)- and Z is optionally substituted phenyl, or Y is -(CH₂)- or -CH(CH₃)- and Z is optionally substituted oxadiazole, particularly 3,5-[1,2,4]-oxadiazole, or Y is -(CH₂)- or -CH(CH₃)- and Z is optionally substituted pyridyl. Within this subclass, also included are those compounds in which R¹ and R² are independently lower alkyl optionally substituted by cycloalkyl, especially n-propyl. In other embodiments are those compounds in which Y is a covalent bond, -(CH₂)- or -CH(CH₃)- and Z is hydrogen, optionally substituted phenyl, or optionally substituted pyridyl, particularly where Y is a covalent bond and Z is hydrogen.

At present, the compounds useful in this invention include, but are not limited to:
1-propyl-8-(1-{[3-(trifluoromethyl)phenyl]-methyl}pyrazol-4-yl)-1,3,7-trihydropurine-2,6-dione;
1-propyl-8-[1-benzylpyrazol-4-yl]-1,3,7-trihydropurine-2,6-dione;
1-butyl-8-(1-{[3-fluorophenyl]methy1}pyrazol-4-yl)-1,3,7-trihydropurine-2,6-dione;
1-propyl-8-[1-(phenylethyl)pyrazo1-4-yl]-1,3,7-trihydropurine-2,6-dione;
8-(1-{[5-(4-chlorophenyl)(1,2,4-oxadiazol-3-yl)]methyl}pyrazol-4-yl)-1-propyl-1,3,7-trihydropurine-2,6-dione;
8-(1-{[5-(4-chlorophenyl)(1,2,4-oxadiazol-3-yl)]methyl}pyrazol-4-yl)-1-butyl-1,3,7-trihydropurine-2,6-dione;
1,3-dipropyl-8-pyrazol-4-yl-1,3,7-trihydropurine-2,6-dione;
1-methyl-3-sec-butyl-8-pyrazol-4-yl-1,3,7-trihydropurine-2,6-dione;
1-cyclopropylmethyl-3-methyl-8-{1-[(3-trifluoromethylphenyl)methyl]pyrazol-4-yl}-1,3,7-trihydropurine-2,6-dione;
1,3-dimethyl-8-{1-[(3-fluorophenyl)methyl]pyrazol-4-yl}-1,3,7-trihydropurine-2,6-dione;
3-methyl-1-propyl-8-{1-[(3-trifluoromethylphenyl)methyl]pyrazol-4-yl}-1,3,7-trihydropurine-2,6-dione;
3-ethyl-1-propyl-8-{1-[(3-trifluoromethylphenyl)methyl]pyrazol-4-yl}-1,3,7-trihydropurine-2,6-dione;
1,3-dipropyl-8-(1-{[3-(trifluoromethyl)phenyl]methyl}pyrazol-4-yl)-1,3,7-trihydropurine-2,6-dione;
1,3-dipropyl-8-{1-[(3-fluorophenyl)methyl]pyrazol-4-yl}-1,3,7-tnhydropurine-2,6-dione;
1-ethyl-3-methyl-8- {1-[(3-fluorophenyl)methyl]pyrazol-4-yl}-1,3,7-trihydropurine-2,6-dione;
1,3-dipropyl-8-{1-[(2-methoxyphenyl)methyl]pyrazol-4-yl}-1,3,7-trihydropurine-2,6-dione;
1,3-dipropyl-8-(1-{[3-(trifluoromethyl)-phenyl]ethyl}pyrazol-4-yl)-1,3,7-trihydropurine-2,6-dione;
1,3-dipropyl-8- {1-[(4-carboxyphenyl)methyl]pyrazol-4-yl}-1,3,7-trihydropurine-2,6-dione;
2-[4-(2,6-dioxo-1,3-dipropyl(1,3,7-trihydropurin-8-yl))pyrazolyl]-2-phenylacetic acid;
8-{4-[5-(2-methoxyphenyl)-[1,2,4]oxadiazol-3-ylmethoxy]phenyl}-1,3-dipropyl-1,3,7-trihydropurine-2,6-dione;
8-{4-[5-(3-methoxyphenyl)-[1,2,4]oxadiazol-3-ylmethoxy]phenyl}-1,3-dipropyl-1,3,7-trihydropurine-2,6-dione;
8-{4-[5-(4-fluorophenyl)-[1,2,4]oxadiazol-3-ylmethoxy]phenyl}-1,3-dipropyl-1,3,7-trihydropurine-2,6-dione:
   1-(cyclopropylmethyl)-8-[1-(2-pyridylmethyl)pyrazol-4-yl]-1,3,7-trihydropurine-2,6-dione;
   1-n-butyl-8- [1-(6-trifluoromethylpyridin-3-ylmethyl)pyrazol-4-yl]-1,3,7-trihydropurine-2,6-dione;
   8-(1-{[3-(4-chlorophenyl)(1,2,4-oxadiazol-5-yl)]methyl}pyrazol-4-yl)-1,3-dipropyl-1,3,7-trihydropurine-2,6-dione;
   1,3-dipropyl-8-[1-({5-[4-(trifluoromethyl)phenyl]isoxazo1-3-yl}methyl)pyrazol-4-yl]-1,3,7-trihydropurine-2,6-dione;
   1,3-dipropyl-8-[1-(2-pyridylmethyl)pyrazol-4-yl]-1,3,7-trihydropurine-2,6-dione;
   3-{[4-(2,6-dioxo-1,3-dipropyl-1,3,7-trihydropurin-8-yl)pyrazolyl]methyl}benzoic acid;
   1,3-dipropyl-8-(1-{[6-(trifluoromethyl)(3-pyridyl)]methyl}pyrazol-4-yl)-1,3,7-trihydropurine-2,6-dione;
   1,3-dipropyl-8-{1-[(3-(1H-1,2,3,4-tetraazol-5-yl)phenyl)methyl]pyrazol-4-yl}-1,3,7-trihydropurine-2,6-dione;
   6-{[4-(2,6-dioxo-1,3-dipropyl-1,3,7-trihydropurin-8-yl)pyrazolyl]methyl}pyridine-2-carboxylic acid;
   3-ethyl-1-propyl-8-[1-(2-pyridylmethyl)pyrazol-4-yl]-1,3,7-trihydropurine-2,6-dione;
   8-(1-{[5-(4-chlorophenyl)isoxazol-3-yl]methyl}pyrazol-4-yl)-3-ethyl-1-propyl-1,3,7-trihydroprine-2,6-dione;
   8-(1-{[3-(4-chlorophenyl)(1,2,4-oxadiazol-5-yl)]methyl}pyrazol-4-yl)-3-ethyl-1-propyl-1,3,7-trihydropurine-2,6-dione;
   3-ethyl-1-propyl-8-(1-{[6-(trifluoromethyl)(3-pyridyl)]methyl}pyrazol-4-yl)-1,3,7-trihydropurine-2,6-dione;
   1-(cyclopropylmethyl)-3-ethyl-8-(1-{[6-(trifluoromethyl)(3-pyridyl)]methyl}pyrazol-4-yl)-1,3,7-trihydropurine-2,6-dione; and
   3-ethyl-1-(2-methylpropyl)-8-(1-{[6-(trifluoromethyl)(3-pyridyl)]methyl}pyrazol-4-yl)-1,3,7-trihydropurine-2,6-dione
   or a pharmaceutically acceptable salt, tautomer, isomer, a mixture of isomers, or prodrug thereof.

It is contemplated that prodrugs of the above-described A_{2B} adenosine receptor antagonists are also useful in the methods of the invention. Exemplary prodrugs are taught in U.S. Patent 7,625,881, which is hereby incorporated by reference in its entirety. Therefore, in one embodiment, the compounds useful in the methods of the invention include prodrugs of Formula III having the formula: 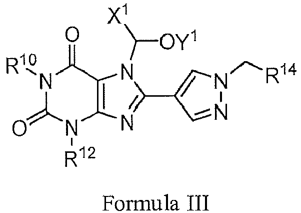 wherein:
- R¹⁰ and R¹²: are independently lower alkyl;
- R¹⁴: is optionally substituted phenyl;
- X¹: is hydrogen or methyl; and
- Y¹: is-C(O)R, in which R is independently optionally substituted lower alkyl, optionally substituted aryl, or optionally substituted heteroaryl; or
- Y¹: is -P(O)(OR⁵)₂, in which R¹⁵ is hydrogen or lower alkyl optionally substituted by phenyl or heteroaryl;
and the pharmaceutically acceptable salts thereof.

One group of compounds of Formula III are those in which R¹⁰ and R¹² are ethyl or n-propyl, especially those compounds in which R¹⁰ is n-propyl and R¹² is ethyl. In another embodiment, R¹⁴ is 3-(trifluoromethyl)phenyl and X¹ is hydrogen.

One subgroup includes those compounds of Formula III in which Y¹ is -C(O)R, particularly those compounds in which R is methyl, ethyl, n-propyl, isopropyl, n-butyl, isobutyl, t-butyl, or n-pentyl, more particularly where R is methyl, n-propyl, or t-butyl. Another subgroup includes those compounds of Formula III in which Y¹ is-P(O)(OR⁵)₂, especially where R¹⁵ is hydrogen.

Compounds or prodrugs of Formula III include, but are not limited to, the following compounds:
[3-ethyl-2,6-dioxo-1-propyl-8-(1-{[3-(trifluoromethyl)phenyl]methyl}pyrazol-4-yl)-1,3,7-trihydropurin-7-yl]methyl acetate;
[3-ethyl-2,6-dioxo-1-propyl-8-(1-{[3-(trifluoromethyl)phenyl]methyl}pyrazol-4-yl)-1,3,7-trihydropurin-7-yl]methyl 2,2-dimethylpropanoate;
[3-ethyl-2,6-dioxo-1-propyl-8-(1-{[3-(trifluoromethyl)phenyl]methyl}pyrazol-4-yl)-1,3,7-trihydropurin-7-yl]methyl butanoate; and
[3-ethyl-2,6-dioxo-1-propyl-8-(1-{[3-(trifluoromethyl)phenyl]methyl}-pyrazol-4-yl)(1,3,7-trihydropurin-7-yl)]methyl dihydrogen phosphate
or pharmaceutically acceptable salts thereof.

In one embodiment, the A_{2B} adenosine receptor antagonist is N-[5-(1-cyclopropyl-2,6-dioxo-3-propyl-2,3,6,7-tetrahydro-1H-purin-8-yl)-pyridin-2-yl]-N-ethyl-nicotinamide (Compound B), also known as ATL-801, having the following chemical formula: 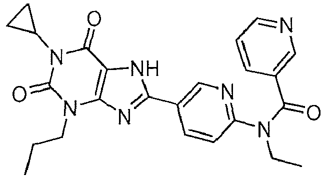 More information about this compound can be found at Kolachala et al. Br J Pharmacol. 155(1):127-37 (2008) and US Patent Application No. 2007-0072843.

In another embodiment, the A_{2B} adenosine receptor antagonist is (2-(4-benzyloxy-phenyl)-N-[5-(2,6-dioxo-1,3-dipropyl-2,3,6,7-tetrahydro-1H-purin-8-yl)-1-methyl-1H-pyrazol-3-yl]-acetamide (Compound C), also known as AS-16, having the following chemical formula: 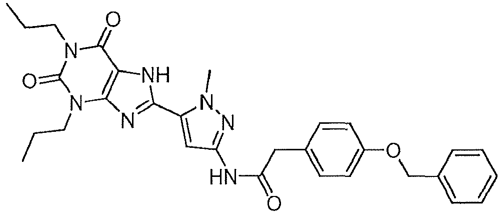 More information about this compound and methods of preparing the compound can be found in Baraldi, P.G. et. al. Journal of Medicinal Chemistry 47:1434-47 (2004).

An A_{2B} adenosine receptor antagonist is any compound that inhibits or otherwise modulates the activity of the A_{2B} receptor. A_{2B} adenosine receptor antagonists are known in the art. For example, several small molecule inhibitors of the receptor have been identified. Exemplary compounds include:

| **Compound Structure** | **Chemical Name** | **Source** |
|---|---|---|
| Compound A | 3-ethyl-1-propyl-8-(1-(3-(trifluoromethyl) benzyl)-1 H-pyrazol-4-yl)-1H-purine-2,6(3H,7H)-dione | U.S. Patent 6,825,349 |
| 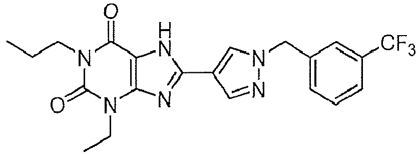 | | |
| 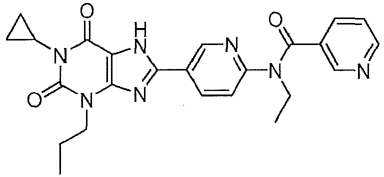 | N-[5-(1-cyclopropyl-2,6-dioxo-3-propyl-2,3,6,7-tetrahydro-1H-purin-8-yl)-pyridin-2-yl]-N-ethyl-nicotinamide | US Published Patent Application 2007/007284 3 |
| 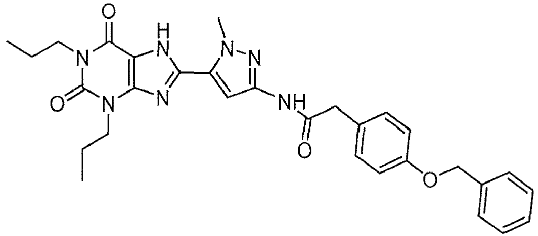 | 2-(4-(benzyloxy)phen yl)-N-(5-(2,6-dioxo-1,3-dipropyl-2,3,6,7-tetrahydro-1H-purin-8-yl)-1-methyl-1H-pyrazol-3-yl)acetamide | US Published Patent Application 2007/007284 3 |

Additional A_{2B} adenosine receptor antagonists are 8-cyclic xanthine derivative, where the cyclic substituent may be aryl, heteroaryl, cycloalkyl, or heterocyclic all of which cyclic groups are optionally substituted as defined above. Examples of 8-cyclic xanthine derivatives may be found throughout the literature, see, *e.g.,* Baraldi, P. et al., "Design, Synthesis, and Biological Evaluation of New 8-Heterocyclic Xanthine Derivatives as Highly Potent and Selective Human A2B adenosine receptor antagonists", J. Med. Chem., (2003), also found in WO02/42298, WO03/02566, WO2007/039297, WO02/42298, WO99/42093, WO2009/118759, and WO2006/044610 which are all incorporated by reference in their entirety.

In one embodiment, the A_{2B} receptor antagonist is a compound having the chemical formula: 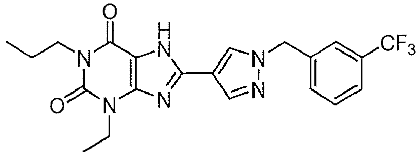 and the name 3-ethyl-1-propyl-8-(1-(3-(trifluoromethyl)benzyl)-1H-pyrazol-4-yl)-1H-purine-2,6(3H,7H)-dione or 3-ethyl-1-propyl-8-(1-((3-(trifluoromethyl)phenyl)methyl)pyrazol-4-yl)-1,3,7-trihydropurine-2,6-dione. It is sometimes referred to throughout as "Compound A" or "CVT-6833." The compound is described in U.S. Patent 6,825,349, which is hereby incorporated by reference in its entirety.

The term "alkyl" refers to a monoradical branched or unbranched saturated hydrocarbon chain having 1, 2, 3, 4, 5, 6, 7, 8, 9, 10, 11, 12, 13, 14, 15, 16, 17, 18, 19 or 20 carbon atoms. This term is exemplified by groups such as methyl, ethyl, n-propyl, iso-propyl, n-butyl, iso-butyl, t-butyl, n-hexyl, n-decyl, tetradecyl, and the like.

The term "substituted alkyl" refers to:
1) an alkyl group as defined above, having 1, 2, 3, 4 or 5 substituents, preferably 1 to 3 substituents, selected from the group consisting of alkenyl, alkynyl, alkoxy, cycloalkyl, cycloalkenyl, acyl, acylamino, acyloxy, amino, aminocarbonyl, alkoxycarbonylamino, azido (-N₃), cyano (CN), halogen, hydroxyl (OH), keto (=O), thiocarbonyl (-C(S)), carboxy (-C(O)OH), carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-, SO-heteroaryl, -SO₂-alkyl, SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2; or
2) an alkyl group as defined above that is interrupted by 1-10 atoms independently chosen from oxygen, sulfur and NRₐ-, where Rₐ is chosen from hydrogen, alkyl, cycloalkyl, alkenyl, cycloalkenyl, alkynyl, aryl, heteroaryl and heterocyclyl. All substituents may be optionally further substituted by alkyl, alkoxy, halogen, CF₃, amino, substituted amino, cyano, or -S(O)ₙR', in which R' is alkyl, aryl, or heteroaryl and n is 0, 1 or 2; or
3) an alkyl group as defined above that has both 1, 2, 3, 4 or 5 substituents as defined above and is also interrupted by 1-10 atoms as defined above.

The term "hydroxyamino" refers to the group -NHOH.

The term "alkoxyamino" refers to the group -NHOR in which R is optionally substituted alkyl.

The term "lower alkyl" refers to a monoradical branched or unbranched saturated hydrocarbon chain having 1, 2, 3, 4, 5, or 6 carbon atoms. This term is exemplified by groups such as methyl, ethyl, n-propyl, iso-propyl, n-butyl, iso-butyl, t-butyl, n-hexyl, and the like.

The term "substituted lower alkyl" refers to lower alkyl as defined above having 1 to 5 substituents, preferably 1, 2, or 3 substituents, as defined for substituted alkyl, or a lower alkyl group as defined above that is interrupted by 1, 2, 3, 4, or 5 atoms as defined for substituted alkyl, or a lower alkyl group as defined above that has both 1, 2, 3, 4 or 5 substituents as defined above and is also interrupted by 1, 2, 3, 4, or 5 atoms as defined above.

The term "alkylene" refers to a diradical of a branched or unbranched saturated hydrocarbon chain, having 1, 2, 3, 4, 5, 6, 7, 8, 9, 10, 11, 12, 13, 14, 15, 16, 17, 18, 19 or 20 carbon atoms, preferably 1-10 carbon atoms, more preferably 1, 2, 3, 4, 5 or 6 carbon atoms. This term is exemplified by groups such as methylene (-CH₂-), ethylene (-CH₂CH₂-), the propylene isomers (e.g., -CH₂CH₂CH₂- and-CH(CH₃)CH₂-) and the like.

The term "alkoxy" refers to the group R'-O-, where R' is optionally substituted alkyl or optionally substituted cycloalkyl, or R' is a group -Y'-Z', in which Y' is optionally substituted alkylene and Z' is optionally substituted alkenyl, optionally substituted alkynyl; or optionally substituted cycloalkenyl, where alkyl, alkenyl, alkynyl, cycloalkyl and cycloalkenyl are as defined herein. Preferred alkoxy groups are optionally substituted alkyl-O- and include, by way of example, methoxy, ethoxy, n-propoxy, iso-propoxy, n-butoxy, tert-butoxy, sec-butoxy, n-pentoxy, n-hexoxy, 1,2-dimethylbutoxy, trifluoromethoxy, and the like.

The term "alkylthio" refers to the group R'-S-, where R' is as defined for alkyl.

The term "alkenyl" refers to a monoradical of a branched or unbranched unsaturated hydrocarbon group preferably having from 2 to 20 carbon atoms, more preferably 2 to 10 carbon atoms and even more preferably 2 to 6 carbon atoms and having 1-6, preferably 1, double bond (vinyl). Preferred alkenyl groups include ethenyl or vinyl (-CH=CH₂), 1-propylene or allyl (-CH₂CH=CH₂), isopropylene (-C(CH₃)=CH₂), bicyclo[2.2.1]heptene, and the like.

The term "substituted alkenyl" refers to an alkenyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -SO₂-alkyl, SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "alkynyl" refers to a monoradical of an unsaturated hydrocarbon, preferably having from 2 to 20 carbon atoms, more preferably 2 to 10 carbon atoms and even more preferably 2 to 6 carbon atoms and having at least 1 and preferably from 1-6 sites of acetylene (triple bond) unsaturation. Preferred alkynyl groups include ethynyl, (-C=CH), propargyl (or prop-1-yn-3-yl, -CH₂C≡CH), and the like.

The term "substituted alkynyl" refers to an alkynyl group as defined above having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -SO₂-alkyl, SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "aminocarbonyl" refers to the group -C(O)NR'R' where each R' is independently hydrogen, alkyl, aryl, heteroaryl, heterocyclyl or where both R' groups are joined to form a heterocyclic group (*e.g.*, morpholino). Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "acylamino" refers to the group -NR'C(O)R' where each R' is independently hydrogen, alkyl, aryl, heteroaryl, or heterocyclyl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "aminocarbonylamino" refers to the group NR'-C(O)-NR'R' where each R' is independently H or as defined for substituted amino.

The term "acyloxy" refers to the groups -O(O)C-alkyl, -O(O)C-cycloalkyl, -O(O)C-aryl, -O(O)C-heteroaryl, and -O(O)C-heterocyclyl. Unless otherwise constrained by the definition, all substituents may be optionally further substituted by alkyl, carboxy, carboxyalkyl, aminocarbonyl, hydroxy, alkoxy, halogen, CF₃, amino, substituted amino, cyano, or -S(O)ₙR', where R' is alkyl, aryl, or heteroaryl and n is 0, 1 or 2.

The term "alkoxycarbonylamino" refers to the group alkyl-O-C(O)-NR' where R' is as defined for acylamino.

The term "aryl" refers to an aromatic carbocyclic group of 6 to 20 carbon atoms having a single ring *(e.g.,* phenyl) or multiple rings *(e.g.,* biphenyl), or multiple condensed (fused) rings (e.g., naphthyl or anthryl). Preferred aryls include phenyl, naphthyl and the like.

The term "arylene" refers to a diradical of an aryl group as defined above. This term is exemplified by groups such as 1,4-phenylene, 1,3-phenylene, 1,2-phenylene, 1,4'-biphenylene, and the like.

Unless otherwise constrained by the definition for the aryl or arylene substituent, such aryl or alkylene groups can optionally be substituted with from 1 to 5 substituents, preferably 1 to 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -SO₂-alkyl, SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "aryloxy" refers to the group aryl-O- wherein the aryl group is as defined above, and includes optionally substituted aryl groups as also defined above. The term "arylthio" refers to the group R'-S-, where R' is as defined for aryl.

The term "amino" refers to the group -NH₂.

The term "substituted amino" refers to the group -NR'R' where each R' is independently selected from the group consisting of hydrogen, alkyl, cycloalkyl, carboxyalkyl (for example, benzyloxycarbonyl), aryl, heteroaryl and heterocyclyl provided that both R' groups are not hydrogen, or a group -Y'-Z', in which Y' is optionally substituted alkylene and Z' is alkenyl, cycloalkenyl, or alkynyl, Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2. When R' is -OH, then this is referred to as hydroxyamino. Similarly, when R' is alkoxy, then this is "alkoxyamino".

The term "carboxyalkyl" refers to the groups -C(O)O-alkyl or -C(O)O-cycloalkyl, where alkyl and cycloalkyl, are as defined herein, and may be optionally further substituted by alkyl, alkenyl, alkynyl, alkoxy, halogen, CF₃, amino, substituted amino, cyano, or -S(O)ₙR', in which R' is alkyl, aryl, or heteroaryl and n is 0, 1 or 2.

The term "cycloalkyl" refers to carbocyclic groups of from 3 to 20 carbon atoms having a single cyclic ring or multiple condensed rings. Such cycloalkyl groups include, by way of example, single ring structures such as cyclopropyl, cyclobutyl, cyclopentyl, cyclooctyl, and the like, or multiple ring structures such as adamantanyl, bicyclo[2.2.1]heptane, 1,3,3-trimethylbicyclo[2.2.1]hept-2-yl, (2,3,3-trimethylbicyclo[2.2.1]hept-2-yl), or carbocyclic groups to which is fused an aryl group, for example indane, and the like.

The term "substituted cycloalkyl" refers to cycloalkyl groups having 1, 2, 3, 4 or 5 substituents, and preferably 1, 2, or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl, -SO-heteroaryl, -SO₂-alkyl, SO₂-aryl and -SO₂-heteroaryl. Unless otherwise constrained by the definition, all substituents may optionally be further substituted by 1, 2, or 3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "cycloalkenyl" refers to a cycloalkyl group, as defined above, having at least one, or from 2-6 points of unsaturation (or double bonds).

The term "halogen" or "halo" refers to fluoro, bromo, chloro, and iodo.

The term "acyl" denotes a group -C(O)R', in which R' is hydrogen, optionally substituted alkyl, optionally substituted cycloalkyl, optionally substituted heterocyclyl, optionally substituted aryl, and optionally substituted heteroaryl.

The term "heteroaryl" refers to a radical derived from an aromatic cyclic group (*i.e.,* fully unsaturated) having 1, 2, 3, 4, 5, 6, 7, 8, 9, 10, 11, 12, 13, 14, or 15 carbon atoms and 1, 2, 3 or 4 heteroatoms selected from oxygen, nitrogen and sulfur within at least one ring. Such heteroaryl groups can have a single ring *(e.g.,* pyridyl or furyl) or multiple condensed rings (*e.g.,* indolizinyl, benzothiazolyl, or benzothienyl). Examples of heteroaryls include, but are not limited to, [1,2,4]oxadiazole, [1,3,4]oxadiazole, [1,2,4]thiadiazole, [1,3,4]thiadiazole, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, and the like as well as N-oxide and N-alkoxy- nitrogen derivatives containing heteroaryl compounds, for example pyridine-N-oxide derivatives.

The term "heteroarylene" refers to a diradical of a heteroaryl group as defined above. This term is exemplified by groups such as 2,5-imidazolene, 3,5-[1,2,4]oxadiazolene, 2,4-oxazolene, 1,4-pyrazolene, and the like. For example, 1,4-pyrazolene is: 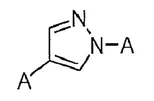 where A represents the point of attachment.

Unless otherwise constrained by the definition for the heteroaryl or heteroarylene substituent, such heteroaryl or heterarylene groups can be optionally substituted with 1 to 5 substituents, preferably 1 to 3 substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -SO₂-alkyl, SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "heteroaryloxy" refers to the group heteroaryl-O-.

The term "heterocyclyl" refers to a monoradical saturated or partially unsaturated group having a single ring or multiple condensed rings, having from 1 to 40 carbon atoms and from 1 to 10 hetero atoms, preferably 1, 2, 3 or 4 heteroatoms, selected from nitrogen, sulfur, phosphorus, and/or oxygen within the ring. Heterocyclic groups can have a single ring or multiple condensed rings, and include tetrahydrofuranyl, morpholino, piperidinyl, piperazino, dihydropyridino, and the like.

Unless otherwise constrained by the definition for the heterocyclic substituent, such heterocyclic groups can be optionally substituted with 1, 2, 3, 4 or 5, and preferably 1, 2 or 3 substituents, selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, heteroarylthio, heterocyclylthio, thiol, alkylthio, aryl, aryloxy, heteroaryl, aminosulfonyl, aminocarbonylamino, heteroaryloxy, heterocyclyl, heterocyclooxy, hydroxyamino, alkoxyamino, nitro, -SO-alkyl, -SO-aryl,-SO-heteroaryl, -SO₂-alkyl, SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR', where R' is alkyl, aryl, or heteroaryl and n is 0, 1 or 2.

The term "heterocyclyloxy" refers to -O-herterocyclyl.

The term "substituted alkylthio" refers to the group -S-substituted alkyl.

The term "heteroarylthio" refers to the group -S-heteroaryl wherein the heteroaryl group is as defined above including optionally substituted heteroaryl groups as also defined above.

The term "heterocyclicthio" refers to the group heterocyclic-S-.

The term "sulfoxide" refers to a group -S(O)R', in which R' is alkyl, aryl, or heteroaryl. "Substituted sulfoxide" refers to a group -S(O)R', in which R' is substituted alkyl, substituted aryl, or substituted heteroaryl, as defined herein.

The term "sulfone" refers to a group -S(O)₂R', in which R' is alkyl, aryl, or heteroaryl. "Substituted sulfone" refers to a group -S(O)₂R', in which R' is substituted alkyl, substituted aryl, or substituted heteroaryl, as defined herein.

The term "aminosulfonyl" refers to the group -SO₂- optionally substituted amino.

The term "keto" refers to a group -C(O)-. The term "thiocarbonyl" refers to a group - C(S)-. The term "carboxy" refers to a group -C(O)-OH.

"Optional" or "optionally" means that the subsequently described event or circumstance may or may not occur, and that the description includes instances where said event or circumstance occurs and instances in which it does not.

The term "compound of Formula I, Formula II, or Formula III" is intended to encompass the compounds of the invention as disclosed, and the pharmaceutically acceptable salts, pharmaceutically acceptable esters, prodrugs, hydrates and polymorphs of such compounds. Additionally, the compounds of the invention may possess one or more asymmetric centers, and can be produced as a racemic mixture or as individual enantiomers or diastereoisomers. The number of stereoisomers present in any given compound of the invention depends upon the number of asymmetric centers present (there are 2ⁿ stereoisomers possible where n is the number of asymmetric centers). The individual stereoisomers may be obtained by resolving a racemic or non-racemic mixture of an intermediate at some appropriate stage of the synthesis, or by resolution of the compound of the invention by conventional means. The individual stereoisomers (including individual enantiomers and diastereoisomers) as well as racemic and non-racemic mixtures of stereoisomers are encompassed within the scope of the present invention, all of which are intended to be depicted by the structures of this specification unless otherwise specifically indicated.

"Isomers" are different compounds that have the same molecular formula.

"Stereoisomers" are isomers that differ only in the way the atoms are arranged in space.

"Enantiomers" are a pair of stereoisomers that are non-superimposable mirror images of each other. A 1:1 mixture of a pair of enantiomers is a "racemic" mixture. The term "(±)" is used to designate a racemic mixture where appropriate.

"Diastereoisomers" are stereoisomers that have at least two asymmetric atoms, but which are not mirror-images of each other.

The absolute stereochemistry is specified according to the Cahn-Ingold-Prelog R-S system. When the compound is a pure enantiomer the stereochemistry at each chiral carbon may be specified by either R or S. Resolved compounds whose absolute configuration is unknown are designated (+) or (-) depending on the direction (dextro- or levorotary) which they rotate the plane of polarized light at the wavelength of the sodium D line.

The term "tautomer" refers to alternate forms of a compound that differ in the position of a proton, such as enol, keto, and imine enamine tautomers, or the tautomeric forms of heteroaryl groups containing a ring atom attached to both a ring NH moiety and a ring =N moiety such as pyrazoles, imidazoles, benzimidazoles, triazoles, and tetrazoles.

The term "prodrug" as used herein, refers to compounds of the present disclosure that include chemical groups which, *in vivo,* can be converted and/or can be split off from the remainder of the molecule to provide for the active drug, a pharmaceutically acceptable salt thereof, or a biologically active metabolite thereof. Suitable groups are well known in the art and particularly include: for the carboxylic acid moiety, a prodrug selected from, *e.g*., esters including, but not limited to, those derived from alkyl alcohols, substituted alkyl alcohols, hydroxy substituted aryls and heteroaryls and the like; amides; hydroxymethyl, aldehyde and derivatives thereof. Structures of such prodrugs can be of Formula III shown below.

Any formula or structure given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorous, fluorine and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶C1 and ¹²⁵I. Various isotopically labeled compounds of the present disclosure, for example those into which radioactive isotopes such as ³H, ¹³C and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients.

The disclosure also included compounds of any formula disclosed herein, in which from 1 to "n" hydrogens attached to a carbon atom is/are replaced by deuterium, in which n is the number of hydrogens in the molecule. Such compounds exhibit increased resistance to metabolism and are thus useful for increasing the half life of any compound when administered to a mammal. See, for example, Foster, "Deuterium Isotope Effects in Studies of Drug Metabolism", Trends Pharmacol. Sci. 5(12):524-527 (1984). Such compounds are synthesized by means well known in the art, for example by employing starting materials in which one or more hydrogens have been replaced by deuterium.

Deuterium labeled or substituted therapeutic compounds of the disclosure may have improved DMPK (drug metabolism and pharmacokinetics) properties, relating to distribution, metabolism and excretion (ADME). Substitution with heavier isotopes such as deuterium may afford certain therapeutic advantages resulting from greater metabolic stability, for example increased in vivo half-life or reduced dosage requirements. An ¹⁸F labeled compound may be useful for PET or SPECT studies. Isotopically labeled compounds of this disclosure and prodrugs thereof can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., ²H or D) may afford certain therapeutic advantages resulting from greater metabolic stability, for example increased *in vivo* half-life or reduced dosage requirements or an improvement in therapeutic index. It is understood that deuterium in this context is regarded as a substituent in the compound of the Formula I, or any Formula disclosed herein,.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium.

In many cases, the compounds of this invention are capable of forming acid and/or base salts by virtue of the presence of amino and/or carboxyl groups or groups similar thereto. The term "pharmaceutically acceptable salt" refers to salts that retain the biological effectiveness and properties of the compounds of Formula I, II, or III, and which are not biologically or otherwise undesirable. Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group.

Specific examples of suitable amines include, by way of example only, isopropylamine, trimethyl amine, diethyl amine, tri(iso-propyl) amine, tri(n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, and the like.

### Nomenclature

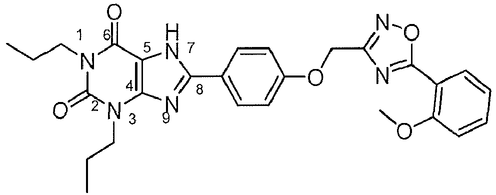

The naming and numbering of the compounds of the invention is illustrated with a representative compound of Formula I in which R¹ is n-propyl, R² is n-propyl, R³ is hydrogen, X is phenylene, Y is -O-(CH₂), and Z is 5-(2-methoxyphenyl)-[1,2,4]-oxadiazol-3-yl, which is named: 8-{4-[5-(2-methoxyphenyl)-[1,2,4]-oxadiazol-3-ylmethoxy]-phenyl]-1,3-dipropyl-1,3,7-trihydropurine-2,6-dione.

### 4. Combination Therapies

Combination therapies are also provided. In one embodiment, the method further comprises administering to the patient an angiotensin-converting enzyme (ACE) inhibitor. Nonlimiting examples of ACE inhibitors include captopril, enalapril, lisinopril, perindopril, ramipril, and the like.

In another embodiment, the method further comprises administering to the patient an angiotensin II receptor antagonists, also known as angiotensin receptor blockers (ARBs). Nonlimiting examples of ARBs include losartan, EXP 3174, candesartan, valsartan, irbesartan, telmisartan, eprosartan, olmesartan, azilsartan, and the like.

A_{2B} adenosine receptor antagonists may be administered in combination with other cardiac fibrosis therapies or agents, including but not limited to Resveratrol (3,5,4'-trihydroxy-trans-stilbene) (Sutra et al., J Agric Food Chem 56(24):11683-11687 (2008)). Resveratrol is a stilbenoid, a type of natural phenol, and a phytoalexin produced naturally by several plants when under attack by pathogens such as bacteria or fungi.

Synergism is contemplated between A_{2B} adenosine receptor antagonists and other cardiac fibrosis therapies due to their respective different therapeutic mechanisms. ACE inhibitors, for instance, block the conversion of angiotensin I to angiotensin II. They, therefore, lower arteriolar resistance and increase venous capacity; increase cardiac output, cardiac index, stroke work, and volume; lower renovascular resistance; and lead to increased natriuresis (excretion of sodium in the urine). In contrast, A_{2B} adenosine receptor antagonists reduce cardiac inflammation and fibrosis. In combination, therefore, an A_{2B} adenosine receptor antagonist and an ACE inhibitor can enhance the cardiac function and reduce the therapeutic dose of each individual agent. The reduction of therapeutic doses, in turn, help reduce or prevent potential adverse events.

In terms of administration, it is contemplated that the two or more agents can be administered simultaneously or sequentially. If the two or more agents are administered simultaneously, they may either be administered as a single dose or as separate doses. Further, it is contemplated that the attending clinician will be able to readily determine the dosage required of the additional agent, the dosing regimen, and the preferred route of administration. Such compositions are prepared in a manner well known in the pharmaceutical art (see, *e.g.,* Remington's Pharmaceutical Sciences, Mace Publishing Co., Philadelphia, PA 17th Ed. (1985) and "Modem Pharmaceutics", Marcel Dekker, Inc. 3rd Ed. (G.S. Banker & C.T. Rhodes, Eds.).

### 5. Administration

The compositions suitable for administration in the invention can be administered to individuals in need thereof orally, intravitreally, topically, sublingually, bucally, nasally, parenterally (e.g., intramuscular), intraperitoneal, intravenous or subcutaneous injection, intracisternally, intravaginally, intraperitoneally, sublingually, bucally, as an oral spray, or a nasal spray. The compositions can be formulated in dosage forms appropriate for each route of administration.

The pharmaceutical compositions can be administered orally, intranasally, ocularly, parenterally or by inhalation therapy, and may take the form of tablets, lozenges, granules, capsules, pills, ampoules, suppositories or aerosol form. They may also take the form of suspensions, solutions and emulsions of the key ingredients in aqueous or nonaqueous diluents, syrups, granulates or powders. In addition to an agent of the present invention, the pharmaceutical compositions can also contain other pharmaceutically active compounds or a plurality of compounds.

In some embodiments, the composition of the invention also referred to herein as the active ingredient, may be administered for therapy by any suitable route including oral, nasal, topical (including transdermal, aerosol, buccal and sublingual), parenteral (including subcutaneous, intramuscular, intravenous and intradermal) and pulmonary. It will also be appreciated that the preferred route will vary with the condition and age of the recipient, and the disease being treated.

### A. Injection

One mode for administration is parental, particularly by injection, such as intravenous (IV) injection. The forms in which the compositions of the present disclosure may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles. Aqueous solutions in saline are also conventionally used for injection, but less preferred in the context of the present disclosure. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

In one aspect, the disclosure provides an IV solution comprising a selected concentration of an A_{2B} adenosine receptor antagonist. Specifically, the IV solution preferably comprises about 1 to about 5000 mg of the A_{2B} adenosine receptor antagonist per milliliter of a pharmaceutically acceptable aqueous solution. Alternatively, the concentration of the A_{2B} adenosine receptor antagonist in the IV solution is from about 5 to about 1000 mg, or alternatively from about 10 to about 800 mg, or alternatively from about 20 to about 800 mg, or alternatively from about 50 to about 700 mg, or alternatively from about 50 to about 500 mg. In another aspect, the concentration of the A_{2B} adenosine receptor antagonist in the IV solution is from about 100 to about 1000 mg, or alternatively from about 100 to about 800 mg, or alternatively from about 100 to about 500 mg, or alternatively from about 200 to about 500 mg, or alternatively from about 200 to about 400 mg, or alternatively from about 200 to about 300 mg. In yet another aspect, the concentration is about 250 mg. In order to allow for the rapid intravenous flow of the A_{2B} adenosine receptor antagonist into the patient, the IV solution preferably contains no viscous components including, by way of example, propylene glycol or polyethylene glycol *(e.g.,* polyethylene glycol 400). It is understood that minor amounts of viscous components that do not materially alter the viscosity may be included in the intravenous formulations of this disclosure. In a particularly preferred embodiment, the viscosity of the IV solution is preferably less than 10 cSt (centistokes) at 20°C, more preferably less than 5 cSt at 20 °C and even more preferably less than 2 cSt at 20 °C.

Sterile injectable solutions are prepared by incorporating the compound of the disclosure in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

### B. Oral Administration

Oral administration is another route for administration of the compounds of the disclosure. Administration may be via capsule or enteric coated tablets, or the like. In making the pharmaceutical compositions that include at least one compound of the disclosure, the active ingredient is usually diluted by an excipient and/or enclosed within such a carrier that can be in the form of a capsule, sachet, paper or other container. When the excipient serves as a diluent, in can be a solid, semi-solid, or liquid material (as above), which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents.

The compositions of the disclosure can be formulated so as to provide quick, sustained or delayed release of the active ingredient after administration to the patient by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Patent Nos. 3,845,770; 4,326,525; 4,902514; and 5,616,345. Another formulation for use in the methods of the present disclosure employs transdermal delivery devices ("patches"). Such transdermal patches may be used to provide continuous or discontinuous infusion of the compounds of the present disclosure in controlled amounts. The construction and use of transdermal patches for the delivery of pharmaceutical agents is well known in the art. *See, e.g.,* U.S. Patent Nos. 5,023,252, 4,992,445 and 5,001,139. Such patches may be constructed for continuous, pulsatile, or on demand delivery of pharmaceutical agents.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient *(e.g.,* a tablet, capsule, ampoule). The compounds of the present disclosure are effective over a wide dosage range and is generally administered in a pharmaceutically effective amount.

In some aspects, for oral administration, each dosage unit contains from 10 mg to 2 g of a compound of the disclosure, or alternatively from 10 to 200 mg, or about 10 mg, 20 mg, 40 mg, 80 mg, or 160 mg. For parenteral administration, the dosage unit can be from 10 to 700 mg of a compound of the disclosure, or about 50-200 mg. It will be understood, however, that the amount of the compound of the disclosure actually administered will be determined by a physician, in the light of the relevant circumstances, including the condition to be treated, the chosen route of administration, the actual compound administered and its relative activity, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present disclosure. When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills of the present disclosure may be coated or otherwise compounded to provide a dosage form affording the advantage of prolonged action, or to protect from the acid conditions of the stomach. For example, the tablet or pill can comprise an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

### C. Inhalation and Insufflation

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner.

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure.

### Formulation Example 1

Hard gelatin capsules containing the following ingredients are prepared:

| **Ingredient** | **(mg/capsule)** |
|---|---|
| Active Ingredient | 30.0 |
| Starch | 305.0 |
| Magnesium stearate | 5.0 |

The above ingredients are mixed and filled into hard gelatin capsules.

### Formulation Example 2

A tablet formula is prepared using the ingredients below:

| **Ingredient** | **(mg/tablet)** |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets.

### Formulation Example 3

A dry powder inhaler formulation is prepared containing the following components:

| **Ingredient** | **Weight %** |
|---|---|
| Active Ingredient | 5 |
| Lactose | 95 |

The active ingredient is mixed with the lactose and the mixture is added to a dry powder inhaling appliance.

### Formulation Example 4

Tablets, each containing 30 mg of active ingredient, are prepared as follows:

| **Ingredient** | **(mg/tablet)** |
|---|---|
| Active Ingredient | 30.0 mg |
| Starch | 45.0 mg |
| Microcrystalline cellulose | 35.0 mg |
| Polyvinylpyrrolidone (as 10% solution in sterile water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50 °C to 60 °C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

### Formulation Example 5

Suppositories, each containing 25 mg of active ingredient are made as follows:

| **Ingredient** | **Amount** |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation Example 6

Suspensions, each containing 50 mg of active ingredient per 5.0 mL dose are made as follows:

| **Ingredient** | **Amount** |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation Example 7

A subcutaneous formulation may be prepared as follows:

| **Ingredient** | **Quantity** |
|---|---|
| Active Ingredient | 5.0 mg |
| Corn Oil | 1.0 mL |

### Formulation Example 8

An injectable preparation is prepared having the following composition:

| **Ingredients** | **Amount** |
|---|---|
| Active ingredient | 2.0 mg/mL |
| Mannitol, USP | 50 mg/mL |
| Gluconic acid, USP | q.s. (pH 5-6) |
| water (distilled, sterile) | q.s. to 1.0 mL |
| Nitrogen Gas, NF | q.s. |

### Formulation Example 9

A topical preparation is prepared having the following composition:

| **Ingredients** | **grams** |
|---|---|
| Active ingredient | 0.2-10 |
| Span 60 | 2.0 |
| Tween 60 | 2.0 |
| Mineral oil | 5.0 |
| Petrolatum | 0.10 |
| Methyl paraben | 0.15 |
| Propyl paraben | 0.05 |
| BHA (butylated hydroxy anisole) | 0.01 |
| Water | q.s. to100 |

All of the above ingredients, except water, are combined and heated to 60 °C with stirring. A sufficient quantity of water at 60 °C is then added with vigorous stirring to emulsify the ingredients, and water then added q.s. 100 g.

### EXAMPLES

The present disclosure is further defined by reference to the following examples. It will be apparent to those skilled in the art that many modifications, both to threads and methods, may be practiced without departing from the scope of the current disclosure.

### Abbreviations

Unless otherwise stated all temperatures are in degrees Celsius (°C). Also, in these examples and elsewhere, abbreviations have the following meanings:

| | | |
|---|---|---|
| µg | = | Microgram |
| µL | = | Microliter |
| µM | = | Micromolar |
| Ado | = | Adenosine |
| AdoR | = | Adenosine Receptor |
| AMI | = | Acute Myocardial Infarction |
| BPM | = | Beats per minute |
| CV | = | Cardiovascular |
| CVD | = | Cardiovascular Diseases |
| EF | = | Ejection Fraction |
| ELISA | = | Enzyme-Linked Immunosorbent Assay |
| g | = | Gram |
| HCF | = | Human Cardiac Fibroblasts |
| IBZ | = | Infarct border zone |
| hr | = | Hour |
| ip or i.p. | = | Intraperitoneal |
| LV | = | Left Ventricular |
| LVEDD | = | Left Ventricular End-Diastolic Diameter |
| LVEF | = | Left Ventricular Ejection Fraction |
| LVESD | = | Left Ventricular End-Systolic Diameter |
| LVESV | = | Left Ventricular End-Systolic Volume |
| LVPWDT | = | LV posterior wall diastolic thickness |
| mg | = | Milligram |
| mL | = | Milliliter |
| mM | = | Millimolar |
| MI | = | Myocardial Infarction |
| MPI | = | Myocardial Performance Index |
| NECA | = | N-ethylcarboxamide adenosine |
| nM | = | *Nanomolar* |
| RVEDA | = | Right Ventricular End-Diastolic Area |
| RT-PCR | = | Reverse Transcription-Polymerase Chain Reaction |
| STEMI | = | ST Segment Elevation Myocardial Infarction |
| SV | = | Stroke Volume |
| TAPSE | = | Tricuspidal Ammlar Plane Systolic Exercusion |

### Methodologies and Reagents

### Cells and reagents

Compound A was synthesized by Gilead Sciences, Inc. (Foster City, California) as provided in U.S. Patent 6,825,349. Other chemical compounds were obtained from Sigma-Aldrich (St. Louis, Missouri).

### Real-time RT-PCR

Real-time RT-PCR was performed as published using Stratagene PCR equipment (La Jolla, California). Zhong H., et al., "A2B adenosine receptors increase cytokine release by bronchial smooth muscle cells, " American Journal of Respiratory Cell and Molecular Biology, 30(1): 118-125 (2004).

### Example 1: Adenosine receptor assays

In order to screen for A_{2B} receptor antagonists, two type of assays are typically used: 1) radioligand binding assay to determine that a given compound could bind to A_{2B} receptor as described below and 2) a functional assay (cAMP assay or others) to determine whether the compound is an agonist (activates the receptor) or an antagonist (inhibits the activation of the receptor).

A radioligand binding assay for A_{2B} adenosine receptor is used to determine the affinity of a compound for the A_{2B} adenosine receptor. Meanwhile, the radioligand binding assays for other adenosine receptors are conducted to determine affinities of the compound for A₁, A_{2A} and A₃ adenosine receptors. The compound should have a higher affinity (at least 3 fold) for A_{2B} receptor than other adenosine receptors.

A cAMP assay for A_{2B} receptor is often used to confirm that the compound is an antagonist and will blocks the A_{2B} receptor-mediated increase in cAMP.

### Radioligand binding for A_{2B} adenosine receptor

Compounds that are putative antagonists of the A_{2B} receptor may be screened for requisite activity based on the following assays. Human A_{2B} adenosine receptor cDNA are stably transfected into HEK-293 cells (referred to as HEK-A2B cells). Monolayer of HEK-A2B cells are washed with PBS once and harvested in a buffer containing 10 mM HEPES (pH 7.4), 10 mM EDTA and protease inhibitors. These cells are homogenized in polytron for 1 minute at setting 4 and centrifuged at 29000 g for 15 minutes at 4 °C. The cell pellets are washed once with a buffer containing 10 mM HEPES (pH 7.4), 1 mM EDTA and protease inhibitors, and are resuspended in the same buffer supplemented with 10% sucrose. Frozen aliquots are kept at -80 °C. Competition assays are started by mixing 10 nM ³H-ZM241385 (Tocris Cookson) with various concentrations of test compounds and 50 µg membrane proteins in TE buffer (50 mM Tris and 1 mM EDTA) supplemented with 1 Unit/mL adenosine deaminase. The assays are incubated for 90 minutes, stopped by filtration using Packard Harvester and washed four times with ice-cold TM buffer (10 mM Tris, 1 mM MgCl₂, pH 7.4). Non specific binding is determined in the presence of 10 µM ZM241385. The affinities of compounds (*i.e.* Ki values) are calculated using GraphPad software.

### Radioligand binding for other adenosine receptors

Human A₁, A_{2A}, A₃ adenosine receptor cDNAs are stably transfected into either CHO or HEK-293 cells (referred to as CHO-A1 HEK-A2A, CHO-A3). Membranes are prepared from these cells using the same protocol as described above. Competition assays are started by mixing 0.5 nM ³H- CPX (for CHO-A1), 2 nM ³H-ZM241385 (HEK-A2A) or 0.1 nM ¹²⁵I-AB-MECA (CHO-A3) with various concentrations of test compounds and the perspective membranes in TE buffer (50 mM Tris and 1 mM EDTA of CHO-A1 and HEK-A2A) or TEM buffer (50 mM Tris, 1 mM EDTA and 10 mM MgCl₂ for CHO-A3) supplemented with 1 Unit/mL adenosine deaminase. The assays are incubated for 90 minutes, stopped by filtration using Packard Harvester and washed four times with ice-cold TM buffer (10 mM Tris, 1 mM MgCl₂, pH 7.4). Non specific binding is determined in the presence of 1 µM CPX (CHO-A1), 1 µM ZM214385 (HEK-A2A) and 1 µM IB-MECA (CHO-A3). The affinities of compounds (*i.e.* Ki values) are calculated using GraphPad software.

### cAMP measurements

Monolayer of transfected cells are collected in PBS containing 5 mM EDTA. Cells are washed once with DMEM and resuspended in DMEM containing 1 Unit/mL adenosine deaminase at a density of 100,000 500,000 cells/mL. 100 µL of the cell suspension is mixed with 25 µL containing various agonists and/or antagonists and the reaction was kept at 37 °C for 15 minutes. At the end of 15 minutes, 125 µL 0.2N HCl is added to stop the reaction. Cells are centrifuged for 10 minutes at 1000 rpm. 100 µL of the supernatant is removed and acetylated. The concentrations of cAMP in the supernatants are measured using the direct cAMP assay from Assay Design.

A_{2A} and A_{2B} adenosine receptors are coupled to Gs proteins and thus agonists for A_{2A} adenosine receptor (such as CGS21680) or for A_{2B} adenosine receptor (such as NECA) increase the cAMP accumulations whereas the antagonists to these receptors prevent the increase in cAMP accumulations-induced by the agonists. A₁ and A₃ adenosine receptors are coupled to Gi proteins and thus agonists for A₁ adenosine receptor (such as CPA) or for A₃ adenosine receptor (such as IB-MECA) inhibit the increase in cAMP accumulations-induced by forskolin. Antagonists to A₁ and A₃ receptors prevent the inhibition in cAMP accumulations.

It is within the skill of one in the art to determine if a compound, based on the above assay protocol, is an antagonist of the A_{2B} receptor. A 3-time, or in certain cases a 10-time selectivity for A_{2B} receptor against other adenosine receptors, can be considered to qualify a compound as a selective A_{2B} receptor antagonist.

### Example 2: A_{2B} Adenosine Receptor Attenuated Cardiac Fibrosis Biomarkers

This example demonstrates that the A_{2B} adenosine receptors (AdoR) is the predominant subtype of AdoRs expressed in primary human cardiac fibroblasts (HCF) and suggests that the A_{2B} AdoR mediates fibrotic response in heart diseases. Thus, an A_{2B} AdoR antagonist can be used to treat cardiac fibrosis.

Expression of AdoR, α-smooth muscle actin and α-1 pro-collagen was determined using real-time RT-PCR. The concentration of IL-6, soluble ST-2 and PAPPA (Pregnancy-associated plasma protein A) in the cell supernatants were measured using ELISA, and the concentration of soluble collagen were determined using Sircol™ collagen assay.

Among the four subtypes of AdoRs, the A_{2B} AdoR was expressed at the highest level in HCF. N-ethylcarboxamide adenosine (NECA), a stable analog of adenosine, significantly increased the release of IL-6 in a concentration-dependent manner, with a maximal increase of 2.4±0.1 fold over the basal level. In addition, NECA (10µM) increased the expression of α-smooth muscle actin and α-1 pro-collagen, and the production of collagen (1.8±0.1 fold induction, from 3.4±0.2 to 6.0±0.4 µg/mL, p<0.05) from HCF. Furthermore, NECA increased the release of two novel biomarkers of cardiovascular diseases (CVD), soluble ST-2 (1.7±0.1 fold induction, from 1.5±0.1 to 2.6±0.1 ng/mL, p<0.05) and PAPPA (4.4±0.6 fold induction, from 1.4±0.5 to 6.2±0.8 ng/mL, p<0.05). The effects of NECA on release of IL-6, collagen, ST-2 and PAPPA and expression of α-smooth muscle actin and α-1 pro-collagen were completely abolished by a selective A_{2B} AdoR antagonist, Compound A (**FIG. 1A-D).**

This example therefore indicates that the A_{2B} AdoR is the predominant subtype of AdoRs expressed in primary human HCF, and activation of this receptor increases the release of IL-6 and production of collagen, expression of fibrotic markers and release of biomarkers of CVD. These findings suggest that A_{2B} AdoR might mediate fibrotic response in heart diseases. Therefore, A_{2B} AdoR antagonists, through inhibiting the activation of A_{2B} AdoR, can be used to treat cardiac fibrosis.

### Example 3: A_{2B} Antagonist Ameliorates Cardiac Remodeling

This example demonstrates that selective blockade of adenosine A_{2B} receptor can ameliorate cardiac remodeling following acute myocardial infarction in the mouse. Adenosine is released in response to tissue injury and promotes hyperemia and inflammation. The proinflammatory effects of adenosine through the A_{2B} receptor provoke further tissue damage. This example tests whether selective blockade of the A_{2B} receptor during acute myocardial infarction would lead to a more favorable cardiac remodeling.

Male ICR mice underwent coronary artery ligation or sham surgery (N=8-10 per group). A selective A_{2B} antagonist, Compound A 4 mg/kg in a formulated dosing suspension, every 12 hours i.p., was given starting immediately after the surgery and continued for 14 days. Transthoracic echocardiography was performed prior to surgery and then 7, 14 and 28 days later. A subgroup of mice was sacrificed 72 hours after surgery and the activity of caspase-1, a key proinflammatory mediator, was measured in the cardiac tissue.

All sham operated mice were alive at 4 weeks, whereas 42% of vehicle-treated mice and 25% of Compound A died during the 4 weeks post-surgery. Treatment with Compound A significantly reduced caspase-1 activity, the end-diastolic diameter and myocardial performance index, and increased LV ejection fraction, as compared to vehicle (FIG. 2).

Therefore, this example demonstrates that selective blockade of adenosine A_{2B} receptor with a selective A_{2B} antagonist, Compound A, limits caspase-1 activation in the heart and leads to a more favorable cardiac remodeling after acute myocardial infarction in the mouse.

### Example 4: A_{2B} Antagonist Attenuates Cardiac Remodeling Following Acute Myocardial Infarction

This example uses an *in vivo* mouse model to demonstrate that selective blockade of A_{2B} AdoR with antagonist reduces caspase-1 activity in the heart leading to a more favorable cardiac remodeling after acute myocardial infarction (AMI).

### Methods

### Experimental AMI model

Adult out-bred male CD1 mice (8-12 weeks of age) were supplied by Harlan Sprague Dawley (Indianapolis, IN). The experiments were conducted under the guidelines of laboratory animals for biomedical research published by National Institutes of Health (No. 85-23, revised 1996). The study protocol was approved by the Virginia Commonwealth University Institutional Animal Care and Use Committee. Experimental AMI was induced by permanent coronary artery ligation in order to induce a large non-reperfused infarct involving approximately 30% of the left ventricle and leading to an ischemic dilated cardiomyopathy (Mezzaroma et al. Proc Natl Acad Sci 2011 - in press and Abbate et al. Circulation 2008;117:2670-83). Briefly, mice were orotracheally intubated under anesthesia (pentobarbital 50 to 70 mg/kg), placed in the right lateral decubitus position, then subjected to a left thoracotomy, pericardiectomy, and ligation of the proximal left coronary artery. The chest was closed and the animals were allowed to recover. The mice surviving surgery were randomly assigned to the different groups of treatment (N=6-15 per group). Sham operations were performed wherein animals underwent the same surgical procedure without coronary artery ligation (N=4-8 per group). A timeline of the protocol of the study is shown in **FIG. 3****.**

### Treatment

The A_{2B} AdoR antagonist, Compound A, was obtained from Gilead Sciences, Foster City, CA. Mice were randomly assigned to treatment with Compound A (4 mg/kg) or matching dose of vehicle administered intraperitoneally (final volume 0.13 mL) every 12 hours for 14 days starting immediately after coronary artery ligation surgery. An additional group of mice received a lower dose of Compound A (2 mg/kg) to explore a dose-response relationship. Two additional groups of mice received Compound A (4 mg/kg) starting 1 hour after surgery to simulate a clinical scenario of treatment delay. An additional group of mice was treated with 0.13 mL of NaCl 0.9% as an additional control, however, because the data of vehicle treatment were not significantly different to those of NaCl treatment, only results of vehicle treatment are included in this example. To simulate a clinically relevant scenario in which drug treatment may occur with some delay after AMI, this example compared treatment with Compound A without delay with groups in which the A_{2B} AdoR antagonist was given with 1 hour of delay.

### Caspase-1 activation

An additional subset of mice was sacrificed 72 hours after surgery (N=4-6 per treatment group). The heart was removed as described above. The tissue activity of caspase-1 was determined by cleavage of a fluorogenic substrate (CaspACE, Promega, Madison, WI) (Abbate et al. Circulation 2008;117:2670-83). After homogenization using RIPA buffer (Sigma Aldrich) containing a cocktail of protease inhibitors (Sigma Aldrich) and centrifugation at 16,000 rpm for 20 minutes, 75 µg of protein from each sample were used for the assay according to the supplier's instructions. Fluorescence was measured after 60 minutes and was expressed as arbitrary fluorescence units produced by one microgram of sample per minute (fluorescence/µg/min) and calculated as fold change compared to the caspase-1 activity in homogenates of the hearts of sham-operated mice.

### Inflammatory infiltrate

In order to quantify the inflammatory infiltrate in the heart during AMI, this example measured CD45 expression (a marker for leukocytes) in the heart using Western Blot. The hearts collected at 72 h after AMI were homogenized in Ripa Buffer (Sigma Aldrich, St Louis, MO) supplemented with a protease inhibitor cocktail (Sigma Aldrich) and centrifuged at 16,200 x g for 20 minutes. Thirty micrograms of each sample were diluted in Laemmli Buffer, denatured for 10 minutes at 96°C and resolved with SDS/PAGE using an 8% acrylamide gel to allow protein separation. The proteins were transferred onto a nitrocellulose membrane. Following saturation with 5% milk in phosphate buffered saline the membrane was incubated with a rat anti-mouse antibody raised against CD45 (R&D system, Minneapolis, MN). To normalize the protein loading a monoclonal antibody for b-actin (Sigma Aldrich) was used. The enhanced-chemiluminescence (ECL) assay and autoradiography were used to detect the bands corresponding to CD45 and b-actin. The band intensity was determined by densitometric analysis using the Scion Image software and the results were expressed as percentage increase in intensity compared to the control sham samples.

### Measurement of circulating levels of cytokines and soluble adhesion molecules

The plasma concentrations of IL-1β and Interleukin-1 (IL-6), Tumor Necrosis Factor-α (TNF-α) and soluble adhesion molecules (E-selectin, Intercellular adhesion molecule-1 [ICAM-1] and vascular cellular adhesion molecule [VCAM]), that are induced by IL-1β were determined at day 28 after surgery using Luminex kits obtained from Millipore (Billerica, MA) according to the manufacturer's instructions. A blood sample was obtained via a direct cardiac puncture immediately prior to killing the animals.

### Echocardiography

All mice underwent transthoracic echocardiography at baseline (before surgery), and at 7, 14 and 28 days after surgery (prior to sacrifice). Echocardiography was performed using the Vevo770 imaging system (VisualSonics Inc, Toronto, Ontario, Canada) with a 30-MHz probe. The heart was visualized in B-mode from parasternal short axis and apical views. This example measured the left ventricular (LV) end-diastolic and end-systolic areas at B-Mode and the LV end-diastolic diameter (LVEDD), LV end-systolic diameters (LVESD), LV anterior wall diastolic thickness (LVAWDT), and LV posterior wall diastolic thickness (LVPWDT) at M-Mode, as previously described (Abbate et al. Circulation 2008;117:2670-83; Toldo et al. PloS One 2011;6:e18102) and according to the American Society of Echocardiography recommendations (Gardin et al. J Am Soc Echocardiogr 2002;15:272-90). LV fractional shortening (FS), LV ejection fraction (EF), LV mass and eccentricity (LVEDD/LVPWDT ratio) were calculated (Abbate et al. Circulation 2008;117:2670-83; Toldo et al. PloS One 2011;6:e18102; Gardin et al. J Am Soc Echocardiogr 2002;15:272-90). The transmitral and left Ventricular out flow tract Doppler spectra were recorded from an apical 4-chamber views, and the myocardial performance index (MPI or Tei index) was calculated as the ratio of the isovolumetric contraction and relaxation time divided by the ejection time (Tei et al. Am J Cardiol 1995;26:357-366). LV stroke volume was calculated using the Velocity-Time Integral (VTI) of the LV outflow tract flow multiplied by the LV outflow tract area, and cardiac output was calculated multiplying LV stroke volume by the heart rate (Abbate et al. Circulation 2008; 117:2670-83; Toldo et al. PloS One 2011;6:e18102; Gardin et al. JAm Soc Echocardiogr 2002;15:272-90). Right Ventricular (RV) enlargement was assessed measuring the RV end-diastolic area in the parasternal short-axis view mid-ventricular section and RV systolic function was estimated using M-Mode and measuring the tricuspidal annular plane systolic excursion (TAPSE) (Toldo et al. PloS One 2011;6:e18102; Gardin et al. J Am Soc Echocardiogr 2002;15:272-90). The investigator performing and reading the echocardiogram was blinded to the treatment allocation.

### Infarct Size Assessment

After the 28-day echocardiogram, all mice were killed with a pentobarbital overdose and/or cervical dislocation. The hearts were explanted and fixed in formalin 10% for at least 48 hours. A transverse section of the median third of the heart was dissected, included in paraffin, cut into 5 µm slides, and stained with Masson's trichrome (Sigma-Aldrich) (Abbate et al. Circulation 2008;117:2670-83). The areas of fibrosis and the whole left ventricle were determined using computer morphometry with the Image Pro Plus 6.0 software.

### Hemodynamic measurements

In a subgroup of mice (N=4 per each group) the LV apex was punctured 1 hour after surgery and a Millar catheter connected to a pressure transducer was inserted to measure LV peak systolic pressure, and heart rate (Toldo et al. PloS One 2011;6:e18102).

### Statistical analyses

Differences between the groups were analyzed using the one-way ANOVA followed by Bonferroni test. Changes in repeated measures of echocardiographic data were analyzed using the random effects ANOVA for repeated-measures to determine the main effect of time, group, and time-by-group interaction. Survival analysis was performed by generating a Kaplan-Meyer survival curve and using logistic regression analysis. Calculations were completed using the SPSS 15.0 package for Windows (SPSS, Chicago, IL).

### Results

### A_{2B} AdoR antagonism had no hemodynamic effects during acute myocardial infarction

As Ado is a vasodilator, and in order to exclude that a difference in remodeling was due to hemodynamic changes secondary to A_{2B} AdoR antagonism, this example measured left ventricular peak systolic pressure (LVPSP) and heart rate (HR) in mice treated with the A_{2B} AdoR antagonist Compound A and those treated with vehicle. LVPSP was significantly reduced 1 hour after coronary artery ligation, but unaffected by treatment (Table 1).

**Table 1. Gross and hemodynamic data**

| ***Group*** | ***Sham*** | ***A_{2B} AdoR antagonist MI*** | ***Vehicle MI*** |
|---|---|---|---|
| **Age (weeks)** | 11±1 | 12±1 | 11±1 |
| **Weight (g)** | 32±1 | 34±1 | 32±1 |
| **LVSP (mmHg)** | 99±3 | 57±9 * | 58±8 * |
| **HR (/min)** | 417±13 | 428±32 | 434±18 |

| | | | |
|---|---|---|---|
| Hemodynamic data were recorded 1 hour after surgery. Abbreviations: A_{2B} AdoR=Adenosine A_{2B} receptor; HR= heart rate; LV= left ventricular; LVSP= peak LV systolic pressure; MI=myocardial infarction * P<0.001 vs sham | | | |

### A_{2B} AdoR antagonism inhibits caspase-1 activation and inflammation

Caspase-1 activation is part of a key proinflammatory mechanism in response to ischemic injury. Treatment with Compound A, prevented caspase-1 activation in the heart during AMI (FIG. 4). The intensity of the leukocyte (CD45+) infiltrate, measured as CD45 expression at Western blot, was also significantly reduced by treatment with Compound A 72 hours after AMI (FIG. 4). Caspase-1 activation results in the processing and release of active IL-1β that is usually present at very low tissue concentration and rapidly amplifies the inflammatory response by inducing the expression of secondary cytokines and adhesion molecules. 1L-1β plasma levels were undetectable in all but 2 mice with AMI whereas plasma levels of secondary cytokines, i.e. IL-6, were increased 28 days after surgery in AMI **(****FIG. 5****).** Treatment with Compound A significantly reduced IL-6, TNF-α, E-selectin, ICAM-1 and VCAM plasma levels **(****FIG. 5****).**

### Effects of A_{2B} AdoR antagonism on survival after coronary artery ligation surgery

None of the sham operated mice died. Half of the vehicle-treated mice (50%) survived to 28 days after coronary artery ligation surgery (P<0.001 vs sham), whereas 75% of mice treated with Compound A were alive (P=0.14).

### Effects of A_{2B} AdoR antagonism on cardiac remodeling

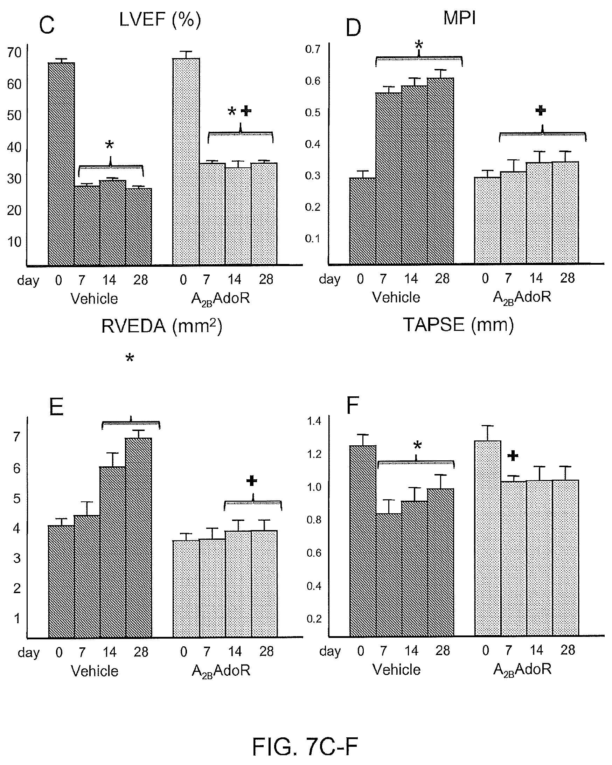

Cardiac remodeling was measured non-invasively using transthoracic echocardiography. Examples of B-Mode and M-Mode recordings are shown in **FIG. 6****.** Administration of Compound A every 12 hours starting at the time of surgery led to a significant attenuation of left and right ventricular enlargement and dysfunction at 7 days, which was maintained at 14 days and also at 28 days, 14 days after the last dose of drug was given **(****FIG. 7****).** At 28 days, LV enlargement after AMI was reduced by approximately 40% by Compound A. LV systolic function was also significantly greater in Compound A group (absolute difference in mean LVEF of 5%). The attenuation in cardiac remodeling was paralleled by preservation of myocardial diastolic/systolic performance (myocardial performance index) **(FIG. 7A-F).** The hearts of mice treated with Compound A also showed less right Ventricular enlargement and dysfunction **(FIG. 7A-F).**

Myocardial ischemia and the accompanying cellular injury are known to cause the release of cell contents triggering a sterile inflammatory response promoting further dysfunction and heart failure. This study shows for the first time that inhibition of Ado binding to the A_{2B}AdoR limits the inflammatory response and leads to a more favorable cardiac remodeling.

Ado is indeed rapidly released during tissue hypoxia and ischemia, and binds rapidly to ubiquitous specific G-protein-coupled receptors (AdoRs). There are 4 subtypes of AdoR. They are: the A₁AdoR, mostly expressed in the heart, regulates electrical conduction; the A₃AdoR is expressed in rodent mast cell, and regulates activation and degranulation in the mouse; whereas the A_{2A}AdoR and A_{2B}AdoR have been linked to vascular tone and inflammation. A_{2A}AdoR are high affinity AdoR expressed on the membrane of various cell types including endothelial cells, leukocytes, and cardiomyocytes. A_{2B}AdoR are low affinity receptors sometimes co-expressed in the same cells expressing A_{2A}AdoR but in a low number and therefore considered to be minimally relevant to Ado signaling in these cells, at least in unstressed conditions. While both A_{2A}AdoR and A_{2B}AdoR G-protein coupled receptors may signal through adenyl cyclase, A_{2B}AdoR also signals through phospholipase C and the small GTP-binding protein p21ras, which is involved in inflammatory signaling involving the p38 mitogen activated phosphokinase (MAPK) and the extracellular signal-regulated kinases (ERK). Importantly, the expression of the A_{2B}AdoR is dependent upon stabilization of the hypoxia inducible factor-α (HIF-α) and hence highly sensitive to hypoxia and inflammation.

Therefore, while in unstressed conditions Ado signaling through the A_{2B}AdoR is likely not significant, in the context of tissue injury the A_{2B}AdoR may play a significant role.

This example shows that selective blockade of the A_{2B}AdoR using Compound A blunted the inflammatory response during the infarction as reflected by a nearly complete reduction in caspase-1 activity, and a significant reduction in infiltrating inflammatory cells early in the course of AMI, and a significant reduction in plasma cytokine and adhesion molecules 28 days after AMI. Caspase-1 is the enzymatically active component of the inflammasome, a macromolecular structure functioning as a 'danger' sensor and involved in the processing of mature IL-β and in cell death. Formation of the inflammasome and activation of caspase-1 in the heart leads to heart failure. The significant reduction in caspase-1 activity in the heart with selective blockade of A_{2B}AdoR is in agreement with the pro-inflammatory signaling of the A_{2B}AdoR. The role of A_{2B}AdoR in myocardial ischemia is however controversial. In models of acute myocardial injury due to ischemia/reperfusion, Ado consistently reproduces the beneficial effects of ischemic preconditioning. The preconditioning-like effects of Ado are eliminated when signaling through the A_{2B}AdoR is disrupted. However, blockade of the A_{2B}AdoR in the absence of ischemic preconditioning had no effects on the heart in such models. This suggests that while it may mediate some aspects of preconditioning, A_{2B}AdoR signaling is not inherently protective, and unlikely to be the sole mediator of preconditioning.

In the current mouse model of large unreperfused myocardial infarction, A_{2B}AdoR antagonism significantly limited left ventricular enlargement and systolic and diastolic dysfunction. The protective effects of the A_{2B}AdoR were independent of an effect on infarct size. This shows that in unreperfused myocardial infarction in the rat, adenosine has no effects on infarct size.

In this model of unreperfused myocardial infarction, cardiac remodeling is global and involves the infarct and border zones as well as the unaffected remote left ventricle and the right ventricle. Blockade of A_{2B}AdoR appeared to have no effects on the infarct size whereas A_{2B}AdoR blockade appeared to protect the border zone and the remote myocardium, as evidenced by an improvement in both left and right ventricular dimensions, and importantly LV function.

The finding of reduced caspase-1 activity in the heart and more favorable cardiac remodeling confirms the central role of caspase-1 in the myocardial response to myocardial ischemia. Overexpression of caspase-1 in the mouse heart leads to larger areas of ischemic damage, more severe cardiac enlargement, and a reduced survival after AMI; whereas caspase-1-deficient mice are protected after AMI.

These data suggest that in infarct healing after myocardial ischemia, the A_{2B}AdoR have a significant pro-inflammatory and deleterious role.

In conclusion, selective blockade of A_{2B} AdoR with Compound A administered after the onset of ischemia reduces caspase-1 activity in the heart and leads to a more favorable cardiac remodeling after AMI in a mouse model of unreperfused myocardial infarction.

### Example 5: Effect of Compound A in a Mouse Model of Myocardial Infarction

The A_{2B} antagonist, Compound A (3 mg/kg/day or 10 mg/kg/day), was given to 6 weeks old ob/ob model mice for 28 days. At the end of the 28-day dosing, expression of a number of inflammatory biomarkers, MCP-1, IL-1b, IL-2 and IL-6, were measured in these mice as compared to control (vehicle).

As shown in **FIG. 8A**-**D**, expression of all four inflammatory biomarkers went down, for two of which, MCP-1 and IL-1b, the reduction for the 10 mg/kg/day group was statistically significant. These data demonstrate that the A_{2B} antagonist Compound A inhibits inflammation in the treated animals.

The effect of Compound A in a mouse model of myocardial infarction was also determined. Male mice underwent permanent coronary artery ligation or sham surgery. Compound A (4 mg/kg) was given i.p. BID starting immediately or 1 hr after the surgery and continued for 14 days. Trans-thoracic ECHO to measure LV end-diastolic/systolic diameter (LVEDD or LVESD), RV end-diastolic area (RVEDA) and myocardial performance index (MPI) was performed prior to surgery and after 7, 14 and 28 days. Analysis of biomarkers of tissue inflammation and injury was conducted on 28 days post-MI.

All sham operated mice were alive at 4 weeks. 42% of vehicle-treated mice with MI were dead during the 4 weeks. In contrast, the morality rate of Compound A-treated mice with MI was only 25%.

Mice with MI developed adverse tissue remodeling and impaired myocardial function as demonstrated by significant increases in LVEDD and LVESD, RVEDA and MPI compared to that in sham mice **(FIG. 9A-D).** Treatment with Compound A immediately or 1hr after the surgery significantly reduced LV/RV dimensions and reduced MPI, suggesting that Compound A inhibited adverse myocardial remodeling and improved myocardial function in mice with MI. Compound A had no effect on myocardial remodeling/function in sham mice.

Plasma biomarkers of inflammation and myocardial injury were analyzed at day 28 post-MI. Plasma concentrations of IL-6, TNF-α and ST2 were significantly increased in mice with MI compared to sham-operated mice. Treatment with Compound A immediately after surgery significantly reduced the concentrations of all plasma biomarkers in mice with MI **(FIG. 10A-C).** Compound A had no effect on plasma biomarkers in sham-operated mice. Soluble cell adhesion molecules (CAMs), such as sE-selectin, sICAM and sVCAM, are important circulating biomarkers for inflammatory processes in cardiovascular diseases. Treatment with Compound A significantly inhibited the increase of plasma levels of soluble cell adhesion molecules in mice with MI **(FIG. 11A-C).**

In summary, these results indicate that treatment with Compound A significantly improves myocardial function, reduces inflammatory circulating biomarkers and thus reduces mortality in the mouse model of post-MI remodeling.

### Example 6: Preclinical Assessment of Compound A

This example includes various preclinical experiments showing the efficacy of Compound A in the care of post- myocardial infarction (MI) animals.

### Rat Model of post-Myocardial Infarction (MI) Remodeling and Ventricular Tachycardia (VT).

The effects of Compound A in a rat model of post-MI remodeling and VT were determined. MI was caused by 25 minutes occlusion at the left anterior descending coronary ligation followed by reperfusion. One week later, rats were administered either vehicle or Compound A (100 mg/kg) once daily by oral gavage. Serial echocardiograms (ECHOs) to measure LV ejection fraction (LVEF), stroke volume and LV end systolic volume (LVESV) were obtained at baseline, 1 week and 5 week post-MI. Electrophysiological studies, optical mapping, histology and biomarker analysis were conducted at 5 week post-MI.

ECHOs obtained at baseline, 1 week and 5 weeks post-MI showed evidence of progressive LV remodeling including significant decrease in LV ejection fraction and stroke volume, and significant increase in LV end systolic volume and in MI rats. In this model, LVEF has been consistently observed to further decline between 1 week and 5 week post-MI. Consistent with this, in the current study LVEF decline from 56.0 ± 2.1% at 1 week post-MI to 40.7 ± 2.2% at 5 weeks post-MI. In contrast, in the animals treated with Compound A, LVEF slightly increased from 53.1 ± 3.2% to 55.6 ± 2.6% **(****FIG. 12B****).** Furthermore, Compound A significantly reduced the increase in LVESV **(****FIG. 12A****).** All these results suggested that Compound A significantly improved post-MI cardiac function and remodeling in rat. In contrast, pirfenidone, which is not an A_{2B} antagonist but has been shown to be able to mitigate left Ventricular fibrosis, did not increase LVESV, but only slightly inhibited its further reduction **(****FIG. 12B****).**

Ventricular tachycardia (VT) is a common cause of mortality in post-MI patients, even with current coronary revascularization treatment. In this rat model of post-MI remodeling, VT was consistently inducible in more than half of the animals. Thus, the effect of Compound A in VT inducibility was determined at 5 weeks post-MI. As shown in Table 3, the rate of VT induction was 54% (6 in 11) in vehicle controls, whereas Compound A significantly reduced VT induction to 9% (1 in 11).

**Table 3. VT inducibility in rat model of post-MI remodeling**

| | **Total N** | **VT** | **No VT** | **% VT** |
|---|---|---|---|---|
| **Placebo** | 11 | 6 | 5 | 54% |
| **Compound A** | 11 | 1 | 10 | 9%* |

| | | | | |
|---|---|---|---|---|
| *, p<0.05 compared to vehicle control using Chi-square test. | | | | |

Abnormal electrical impulse conduction in the infarct border zone (IBZ) is important in the pathogenesis of post-MI arrhythmias. As shown in the conduction vector maps **(FIG. 13A-B),** the vehicle control group has much slower conduction velocity than the Compound A treated animals. To quantify the impact of Compound A on conduction properties, conduction velocities (CVs) were measured for infarct, border, and normal zones of LV myocardium. CVs in normal zones were fastest among all zones and were similar between placebo and Compound A groups **(****FIG. 14A****).** Furthermore, CVs in infarct zones of placebo control group were slowest among all zones and were significantly improved by Compound A **(FIG. 14C).** Finally, CVs in IBZs of both groups were in between those of non-infarct and infarct zones. However, CVs in IBZs of the Compound A group were significantly faster than those in placebo control IBZs **(****FIG. 14B****).**

Abnormal conduction in the IBZ is due to tissue remodeling. Excessive fibrosis in the IBZ is an important substrate for VT vulnerability. As shown in **FIG**. **15A-B,** analysis of vehicle control IBZs revealed more heterogeneous, patchy projections of fibrosis from infarct zones into IBZs. These fibrotic projections are fewer in the IBZs of Compound A treated group, suggesting that Compound A inhibited fibrosis in the IBZ associated with improved conduction velocity, which may explain the markedly reduced inducibility of VT.

Plasma biomarkers of inflammation (IL-6), and tissue injury (BNP and (PAI-1) were analyzed at 5 week post-MI. Plasma concentrations of IL-6 and PAI-1 were significantly increased in post-MI rats compared to normal rats. Treatment of Compound A significantly reduced all these plasma biomarkers in post-MI rats. In addition, there was a trend for Compound A to inhibit the increase of BNP (P=0.16) in post-MI rats. **(FIG. 16A-C)**

Further, this example used a clinically relevant treatment plan in which drug was administered after reperfusion therapy and the LV dysfunction has already been established. Moreover, the effects of treatment were measured using clinically relevant endpoints such as left ventricular end systolic volume and LVEF.

The results of the study in the rat model of post-MI remodeling indicate that treatment of Compound A significantly improves myocardial function and reduces VT vulnerability. This is likely due to reducing inflammatory mediators and inhibiting fibrosis in the border zone of ischemia myocardium.

### Example 7: Other A_{2B} Adenosine Receptor Antagonists

This example uses human cardiac myocytes to test two other A_{2B} adenosine receptor antagonists on their effect on NECA-induced IL-6 release.

Human cardiac myocytes (HCM) were treated with NECA alone, or in combination with Compound A, Compound B (N-[5-(1-cyclopropyl-2,6-dioxo-3-propyl-2,3,6,7-tetrahydro-1H-purin-8-yl)-pyridin-2-yl]-N-ethyl-nicotinamide, also known as ATL-801) or Compound C (2-(4-benzyloxy-phenyl)-N-[5-(2,6-dioxo-1,3-dipropyl-2,3,6,7-tetrahydro-1H-purin-8-yl)-1-methyl-1H-pyrazol-3-yl]-acetamide). As shown in **FIG. 17****,** NECA significantly increased IL-6 release from human cardiac myocytes (HCM). The effect of NECA on HCM, however, was completely abolished by each of the tested A_{2B} AdoR antagonists, Compound A, B and C.

This example, therefore, demonstrates that A_{2B} AdoR antagonists, in general, have the capability to inhibit NECA-induced IL-6 release from human cardiac myocytes.

It will be appreciated that those skilled in the art will be able to devise various arrangements which, although not explicitly described or shown herein, embody the principles of the disclosure and are included within its spirit and scope. Furthermore, all conditional language recited herein is principally intended to aid the reader in understanding the principles of the disclosure and the concepts contributed by the inventors to furthering the art, and are to be construed as being without limitation to such specifically recited conditions. Moreover, all statements herein reciting principles, aspects, and embodiments of the disclosure are intended to encompass both structural and functional equivalents thereof. Additionally, it is intended that such equivalents include both currently known equivalents and equivalents developed in the future, *i.e*., any elements developed that perform the same function, regardless of structure. The scope of the present disclosure, therefore, is not intended to be limited to the exemplary embodiments shown and described herein. Rather, the scope and spirit of present disclosure is embodied by the appended claims.

Further aspects relating to the invention are set out in the following numbered clauses:
1. A method of treating heart failure and/or arrhythmia in a patient that has suffered myocardial infarction (MI), comprising administering to the patient a therapeutically effective amount of an A_{2B} adenosine receptor antagonist.
2. The method of clause 1, wherein death or hospitalization is reduced by treatment of the heart failure and/or arrhythmia.
3. A method of reducing the progression of heart failure in a patient that has suffered myocardial infarction (MI), comprising administering to the patient a therapeutically effective amount of an A_{2B} adenosine receptor antagonist.
4. A method of reducing arrhythmia in a patient that has suffered myocardial infarction (MI), comprising administering to the patient a therapeutically effective amount of an A_{2B} adenosine receptor antagonist.
5. A method of reducing the incidence of sudden cardiac death in a patient that has suffered myocardial infarction (MI), comprising administering to the patient a therapeutically effective amount of an A_{2B} adenosine receptor antagonist.
6. A method of increasing the left ventricle ejection fraction (LVEF) in a patient that has suffered myocardial infarction (MI), comprising administering to the patient a therapeutically effective amount of an A_{2B} adenosine receptor antagonist.
7. A method of inhibiting left ventricle enlargement in a patient that has suffered myocardial infarction (MI), comprising administering to the patient a therapeutically effective amount of an A_{2B} adenosine receptor antagonist.
8. A method of reducing left ventricle end systolic volume in a patient that has suffered myocardial infarction (MI), comprising administering to the patient a therapeutically effective amount of an A_{2B} adenosine receptor antagonist.
9. A method of reducing left ventricle end diastolic volume in a patient that has suffered myocardial infarction (MI), comprising administering to the patient a therapeutically effective amount of an A_{2B} adenosine receptor antagonist.
10. A method of ameliorating left ventricle dysfunction in a patient that has suffered myocardial infarction (MI), comprising administering to the patient a therapeutically effective amount of an A_{2B} adenosine receptor antagonist.
11. A method of improving myocardial contractibility in a patient that has suffered myocardial infarction (MI), comprising administering to the patient a therapeutically effective amount of an A_{2B} adenosine receptor antagonist.
12. A method of reducing the release of IL-6, TNFa, BNP, or ST2 (suppression of tumorigenicity 2) from a cardiac cell in a patient that has suffered myocardial infarction (MI), comprising administering to the patient a therapeutically effective amount of an A_{2B} adenosine receptor antagonist.
13. The method of any one of clauses 1-12, wherein the A_{2B} adenosine receptor antagonist is a 8-cyclic xanthine derivative.
14. The method of any one of clauses 1-12, wherein the A_{2B} adenosine receptor antagonist is a compound of Formula I or II: 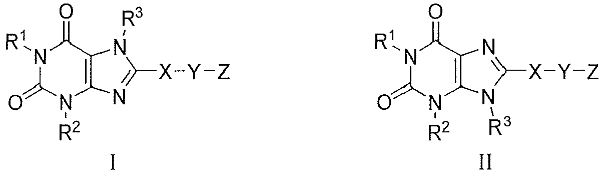 wherein:
   - R¹ and R²: are independently chosen from hydrogen, optionally substituted alkyl, or a group -D-E, in which D is a covalent bond or alkylene, and E is optionally substituted alkoxy, optionally substituted cycloalkyl, optionally substituted aryl, optionally substituted heteroaryl, optionally substituted heterocyclyl, optionally substituted alkenyl or optionally substituted alkynyl;
   - R³: is hydrogen, optionally substituted alkyl or optionally substituted cycloalkyl;
   - X: is optionally substituted arylene or optionally substituted heteroarylene;
   - Y: is a covalent bond or alkylene in which one carbon atom can be optionally replaced by -O-, -S-, or -NH-, and is optionally substituted by hydroxy, alkoxy, optionally substituted amino, or -COR, in which R is hydroxy, alkoxy or amino; and
   - Z: is optionally substituted monocyclic aryl or optionally substituted monocyclic heteroaryl; or
   - Z: is hydrogen when X is optionally substituted heteroarylene and Y is a covalent bond;
   or a pharmaceutically acceptable salt, tautomer, isomer, a mixture of isomers, or prodrug thereof.
15. The method of any one of clauses 1-12, wherein the A_{2B} adenosine receptor antagonist is a compound having the chemical formula: 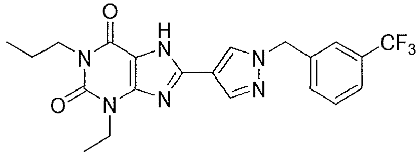 and the name 3-ethyl-1-propyl-8-(1-(3-(trifluoromethyl)benzyl)-1H-pyrazol-4-yl)-1H-purine-2,6(3H,7H)-dione or 3-ethyl-1-propyl-8-(1-((3-(trifluoromethyl)phenyl)methyl)pyrazol-4-yl)-1,3,7-trihydropurine-2,6-dione or a pharmaceutically acceptable salt, tautomer, isomer, or a mixture of isomers thereof.
16. The method of any one of clauses 1-12, wherein the A_{2B} adenosine receptor antagonist is N-[5-(1-cyclopropyl-2,6-dioxo-3-propyl-2,3,6,7-tetrahydro-1H-purin-8-yl)-pyridin-2-yl]-N-ethyl-nicotinamide.
17. The method of any one of clauses 1-12, wherein the A_{2B} adenosine receptor antagonist is (2-(4-benzyloxy-phenyl)-N-[5-(2,6-dioxo-1,3-dipropyl-2,3,6,7-tetrahydro-1H-purin-8-yl)-1-methyl-1H-pyrazol-3-yl]-acetamide.
18. The method of any one of clauses 1-17, wherein the MI is acute MI.
19. The method of clause 18, wherein the MI is ST elevation MI (STEMI) or non-ST elevation MI (NSTEMI).
20. The method of any one of clauses 1-19, wherein the patient is hemodynamically stable.
21. The method of any one of clauses 1-20, wherein the administration of the A_{2B} adenosine receptor antagonist starts during the MI or immediately following the MI.
22. The method of any one of clauses 1-20, wherein the administration of the A_{2B} adenosine receptor antagonist starts after at least about 24 hours following the MI.
23. The method of clause 22, wherein the administration of the A_{2B} adenosine receptor antagonist starts after at least about 3 days following the MI.
24. The method of clause 22, wherein the administration of the A_{2B} adenosine receptor antagonist starts after at least about 5 days following the MI.
25. The method of clause 22, wherein the administration of the A_{2B} adenosine receptor antagonist starts after at least about 7 days following the MI.
26. The method of any one of clauses 1-25, further comprising administering to the patient an angiotensin-converting enzyme (ACE) inhibitor.
27. The method of any preceding clause, wherein the ACE inhibitor is selected from the group consisting of captopril, enalapril, lisinopril, perindopril and ramipril.
28. The method of any preceding clause, wherein the patient is human.
29. The of any preceding clause, wherein the administration is systemic, oral, intravenous, intramuscular, intraperitoneal or by inhalation.

## Claims

1. A compound having the chemical formula: 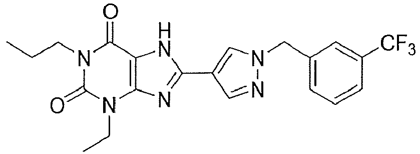 or a pharmaceutically acceptable salt, for use in treating heart failure and/or arrhythmia, reducing the progression of heart failure, reducing the incidence of sudden cardiac death, increasing the left ventricle ejection fraction (LVEF), inhibiting left ventricle enlargement, reducing left ventricle end systolic volume, reducing left ventricle end diastolic volume, ameliorating left ventricle dysfunction, or improving myocardial contractibility in a patient that has suffered myocardial infarction (MI).

2. The compound for use of claim 1, wherein the MI is acute MI.

3. The compound for use of claim 2, wherein the MI is ST elevation MI (STEMI) or non-ST elevation MI (NSTEMI).

4. The compound for use of any of claims 1 to 3, wherein the patient is hemodynamically stable.

5. The compound for use of any of claims 1 to 4, wherein the use comprises administration of the compound to the patient and the administration starts during the MI or immediately following the MI.

6. The compound for use of any of claims 1 to 4, wherein the use comprises administration of the compound to the patient and the administration starts after at least about 24 hours following the MI.

7. The compound for use of claim 6, wherein the administration of the compound starts after at least about 3 days following the MI.

8. The compound for use of claim 6, wherein the administration of the compound starts after at least about 5 days following the MI.

9. The compound for use of claim 6, wherein the administration of the compound starts after at least about 7 days following the MI.

10. The compound for use of any of claims 1 to 9, wherein the use further comprises administering to the patient an angiotensin-converting enzyme (ACE) inhibitor.

11. The compound for use of claim 10, wherein the ACE inhibitor is selected from the group consisting of captopril, enalapril, lisinopril, perindopril and ramipril.

12. The compound for use of any preceding claim, wherein the compound is administered to the patient via a route selected from systemic, oral, intravenous, intramuscular, intraperitoneal or by inhalation.
